# EUROPÄISCHE PATENTSCHRIFT

(11) **EP 1 670 919 B1**
(45) Veröffentlichungstag und Bekanntmachung des Hinweises auf die Patenterteilung: **09.04.2008**
(21) Anmeldenummer: 04802618.1
(22) Anmeldetag: 01.10.2004
(51) Int. Cl.: C12N 15/63, A61K 48/00, C12N 15/10

(54) **VERFAHREN ZUR HERSTELLUNG EINES ZELL- UND/ODER GEWEBE- UND/ODER KRANKHEITSPHASEN-SPEZIFISCHEN ARZNEIMITTELS**
METHOD FOR THE PRODUCTION OF A CELL AND/OR TISSUE AND/OR DISEASE PHASE SPECIFIC MEDICAMENT
PROCEDE POUR PRODUIRE UN MEDICAMENT SPECIFIQUE DE CELLULES ET/OU DE TISSUS ET/OU DE PHASES DE MALADIES

(30) Priorität: 02.10.2003 DE 10346487
(43) Veröffentlichungstag der Anmeldung: 21.06.2006
(60) Teilanmeldung: 07013576.9
(73) Patentinhaber: STERNA BIOLOGICALS GmbH & Co. KG, 35043 Marburg (DE)
(72) Erfinder: SEL, Serdar, 35043 Marburg (DE); RENZ, Harald, 35043 Marburg-Cappel (DE)
(74) Vertreter: Meyer-Dulheuer, Karl-Hermann
(86) Internationale Anmeldenummer: PCT/DE2004/002197
(87) Internationale Veröffentlichungsnummer: WO 2005/033314

(56) Entgegenhaltungen:
- WO-A-00/42173
- WO-A-00/51621
- WO-A-01/11023
- SANTORDO S W ET AL: "A GENERAL PURPOSE RNA-CLEAVING DNA ENZYME" PROCEEDINGS OF THE NATIONAL ACADEMY OF SCIENCES OF USA, NATIONAL ACADEMY OF SCIENCE. WASHINGTON, US, Bd. 94, April 1997 (1997-04), Seiten 4262-4266, XP001009844 ISSN: 0027-8424
- SUN L Q ET AL: "Catalytic nucleic acids: From lab to applications" September 2000 (2000-09), PHARMACOLOGICAL REVIEWS, WILLIAMS AND WILKINS INC., BALTIMORE, MD,, US, PAGE(S) 325-347 , XP002272275 ISSN: 0031-6997 Seite 330 - Seite 337
- IMAGAWA S ET AL: "NEGATIVE REGULATION OF THE ERYTHROPOIETIN GENE EXPRESSION BY THE GATA TRANSCRIPTION FACTORS" 15. Februar 1997 (1997-02-15), BLOOD, W.B. SAUNDERS, PHILADELPHIA, VA, US, PAGE(S) 1430-1439 , XP000965159 ISSN: 0006-4971 Seite 1430, rechte Spalte, Absatz 2

## Beschreibung

Die vorliegende Erfindung betrifft ein Verfahren zur Herstellung eines Zell und/oder Gewebe- und/oder Krankheitsphasen-spezifischen Arzneimittels, das zur Behandlung von chronischen Entzündungen geeignet ist.

### Hintergrund der Erfindung

Chronische Entzündungen stellen einen zunehmend großen medizinischen Problemkreis mit hohem sozioökonomischen Impakt dar. Hierzu zählen insbesondere folgende Erkrankungsgruppen:
- Autoimmunerkrankungen und Erkrankungen des rheumatischen Formenkreises (Manifestationen an u.a. Haut, Lunge, Niere, Gefäßsystem, Nervensystem, Bindegewebe, Bewegungsapparat, endokrinem System)
- Allergische Soforttypreaktionen und Asthma
- Chronisch-obstruktive Lungenerkrankungen (COPD)
- Arteriosklerose
- Psoriasis und Kontaktekzem
- Chronische Abstoßungsreaktionen nach Organ-, Knochenmarkstransplantation

Viele dieser Erkrankungen zeigen in den letzten Dekaden eine ansteigende Prävalenz nicht nur in den Industrienationen, sondern zum Teil weltweit. So leiden in Europa, Nordamerika, Japan und Australien mittlerweile über 20% der Bevölkerung an allergischen Erkrankungen und Asthma. Chronisch-obstruktive Lungenerkrankungen sind zurzeit die fünfthäufigste Todesursache weltweit und werden nach Berechnungen der WHO im Jahre 2020 die dritthäufigste Todesursache darstellen. Arteriosklerose mit den Folgeerkrankungen Herzinfarkt, Schlaganfall und peripherer arterielle Verschlusskrankheit nehmen in der Morbiditäts- und Mortalitätsstatistik weltweit eine führende Position ein. Psoriasis und Kontaktekzem sind zusammen mit der Neurodermitis die häufigsten chronischen Entzündungserkrankungen an der Haut überhaupt.

Aufgrund von bis heute nur unzureichend verstandenen Wechselwirkungen zwischen Umweltfaktoren und einer genetischen Disposition kommt es zu nachhaltigen Fehlregulationen des Immunsystems. Hierbei lassen sich für diese unterschiedlichen Erkrankungen folgende gemeinsame Prinzipien feststellen:
(A) Es kommt zur Entwicklung einer überschießenden Immunantwort gegen normalerweise für den Menschen harmlose Antigene. Diese Antigene können Bestandteile der Umwelt sein (z. B. Allergene, wie Pollen, Tierhaare, Nahrungsmittel, Milben, chemische Substanzen wie Konservierungsstoffe, Farbstoffe, Reinigungsmittel). In diesen Fällen entwickelt sich bei den Patienten eine allergische Reaktion. Im Falle von z.B. Aktiv- und Passiv-Zigarettenrauchern kommt es zu chronisch-obstruktiven Lungenerkrankungen (COPD). Andererseits kann das Immunsystem aber auch gegen Komponenten des eigenen Organismus reagieren, diese als fremd erkennen und eine Entzündungsreaktion dagegen in Gang setzen. In diesen Fällen entwickelt sich eine Autoimmunerkrankung. In jedem Falle werden harmlose, nicht-toxische Antigene fälschlicherweise als fremd bzw. gefährlich erkannt und eine unangemessene Entzündungsreaktion in Gang gesetzt.
(B) Die Erkrankungen verlaufen in Phasen zu denen die Initiation, Progression, also Fortschreiten der Entzündungsreaktion, und die damit assoziierte Destruktion und der Umbau mit Verlust von Organ-Funktionalität (sogenanntes Remodeling) zählen.
(C)Die Erkrankungen zeigen Patienten-spezifische sub-phänotypische Ausprägungsmerkmale.
(D)An der Initiation, Aufrechterhaltung und den Destruktions- und Umbauprozessen sind Komponenten der angeborenen und erworbenen Immunität nachhaltig beteiligt. Unter dem Einfluss der angeborenen Immunität (wichtige Komponenten: Antigen-präsentierende-Zellen mit ihren diversen Populationen und das Komplementsystem) kommt es zu Aktivierung und Differenzierung der Zellen des adaptiven Immunsystems (wichtige Komponenten: T- und B-Lymphozyten). Die T-Zellen übernehmen zentrale Funktionen im weiteren Verlauf indem sie in hoch-spezialisierte Effektoren differenzieren. Hierbei aktivieren und erwerben sie bestimmte Effektormechanismen, zu denen insbesondere folgende Funktionen zählen: Antikörperproduktion, Kontrolle der Funktionalität von Effektorzellen des Immunsystems (wie z. B. neutrophile-, basophile-, eosinophile Granulozyten), Rückkopplung auf Funktionen des angeborenen Immunsystems, Beeinflussung der Funktionalität von nichthämatopoetischen Zellen wie z. B. Epithel, Endothel, Bindegewebe, Knochen und Knorpel und vor allem neuronale Zellen. Hier kommt es zu einer besonderen Wechselwirkung zwischen Immun- und Nervensystem, aus dem sich das Konzept der Neuro-immunologischen Interaktion bei chronischen Entzündungen entwickelt hat

Aufgrund der Komplexität und Vielschichtigkeit der Krankheitsbilder, die mit chronischen Entzündungen einhergehen, müssen an ein optimales Arzneimittel zur Behandlung der Krankheiten folgende Anforderungen gestellt werden:
(1) Erkrankungen manifestieren sich in Patienten-spezifischen (Sub)-Phänotypen. Arzneimittel müssen daher eine hohe Patienten- bzw. Fallspezifität aufweisen.
(2) Erkrankungen verlaufen in Stadien und Phasen. Arzneimittel müssen daher eine Stadien- bzw. Phasenspezifität besitzen.
(3) Die Erkrankungen werden von unterschiedlich spezialisierten Zellen reguliert. Die Arzneimittel müssen daher eine Zell-spezifische Intervention bewirken.
(4) Die Erkrankungen manifestieren sich an unterschiedlichen Organen und Kompartimenten. Die Arzneimittel müssen daher eine Kompartiment- bzw. Organ-Spezifität besitzen.
(5) Arzneimittel müssen für eine Langzeittherapie geeignet sein. So müssen Reaktionen des Immunsystems gegen die Arzneimittel verhindert werden.
(6) Das Nebenwirkungsprofil der Arzneimittel muss in medizinischer und ethischer Relation zu Schweregrad, Prognose und Verlauf der Erkrankungen in einem akzeptablen Verhältnis stehen.

Keine der heute verfügbaren, etablierten Therapien gegen chronische Entzündungen erfüllt diese Kriterien optimal. Bekannt sind aus der DE 695 11 245 T2 die Behandlung mit Immunglobulin A und aus der DE 695 18 667 T2 die Hemmung der Phospholipase A₂ (PLA₂) und /oder Coenzym-A-unabhängigen Transacylase (CoA-IT). Im Mittelpunkt der heute etablierten Therapiekonzepte stehen für diese Erkrankung die unspezifische anti-inflammatorische Therapie, sowie die Immunsuppression. So sind viele der eingesetzten unspezifischen anti-inflammatorisch wirkenden Substanzen wie Ibuprofen, Acetylsalicylsäure und Paracetamol entweder nicht wirksam genug oder mit einer hohen Rate unerwünschter Nebenwirkungen behaftet. Steroide haben dagegen zwar eine höhere Wirkungspotenz, sind aber ihrerseits mit schwerwiegenden Nebenwirkungen wie Hypertonus, Diabetes und Osteoporose behaftet. Immunsuppressive Medikamente der neueren Generation wie z. B. Cyclosporin und Tacrolimus zeigen Hepato- und Nephrotoxizität.

Diese Situation hat zur Suche und klinischen Erprobung einer Vielzahl von neueren Molekülen geführt, die spezifischer in die immunologischen und zellbiologischen Fehlregulationen eingreifen sollen. Hierzu zählen Zytokine, Zytokin-Rezeptoren und Anti-Zytokine. Probleme, die mit diesen neueren therapeutischen Einsätzen verbunden sind, schließen mangelnde Zell- und Organ-Spezifität. Entwicklung von unerwünschten Immunreaktionen gegen diese Moleküle, sowie eine mangelnde Wirksamkeit bei verschiedenen Phänotypen ein.

Es wird in neuerer Zeit versucht, eine neue Klasse katalytischer Moleküle, die so genannten "DNAzyme" (Santoro, 1997) als therapeutische Agenzien zur Inaktivierung von Genen einzusetzen, deren Expression Krankheiten verursacht. DNAzyme sind Einzelstrang-Moleküle, die prinzipiell an komplementäre Bereiche der RNA binden können und diese durch Spaltung inaktivieren. Der spezifische Einsatz von DNAzymen als therapeutische Agenzien setzt allerdings voraus, dass die krankheitsverursachenden Gene und deren mRNA genauestens bekannt sind. Dies ist bislang nur bei wenigen Erkrankungen der Fall.

Das in der WO 01/11023A1 beschriebene DNAzym bindet RelA (p65) mONA und ist damit gegen den Transkriptionsfaktor NF-κB gerichtet, in der WO 00/42173 ist ein EGR-1 mRNA bindendes DNAzym offenbart. Die W099/50452 offenbart ein 10-23 DNAzym, das in einem diagnostischen Verfahren zum Auffinden von Nukleinsäure-Mutationen verwendet werden kann.
Keine der derzeit bekannten Antisense-Moleküle und DNAzyme können zur Herstellung eines Arzneimittels zur Behandlung von chronischen Entzündungen in Patienten verwendet werden.

### Aufgabe der Erfindung

Aufgabe der vorliegenden Erfindung ist es, Zell- und/oder Gewebe- und/oder Krankheitsphasen-spezifische Arzneimittel bereitzustellen, die zur funktionellen Inaktivierung von Ribonukleinsäure-Molekülen von Transkriptionsfaktoren und Faktoren der Signaltransduktionswege, deren Expression an der Entstehung von chronischen Entzündungsreaktionen und Autoimmunerkrankungen beteiligt ist, führen und die zur Behandlung von chronischen Entzündungsreaktionen und Autoimmunerkrankungen geeignet sind, wobei die geschilderten Nachteile im Stand der Technik beseitigt werden.
Darüber hinaus ist es Aufgabe der Erfindung, ein Verfahren zur Herstellung Zell und/oder Gewebe- und/oder Krankheitsphasen-spezifischer Arzneimittel bereitzustellen, das Ribonukleinsäure-Moleküle von Transkriptionsfaktoren und von Faktoren der Signaltransduktionswege, deren Expression an der Entstehung von chronischen Entzündungsreaktionen und Autoimmunerkrankungen beteiligt ist, identifiziert und sie in Zielzellen funktionell inaktiviert.

Die Aufgabe wird erfindungsgemäß durch spezifische DNAzyme gemäß den Ansprüchen 1 bis 5, ein Verfahren gemäß Anspruch 6 und ein Arzneimittel sowie dessen Verwendung gemäß den Ansprüchen 7 bis 9 gelöst.
Der Vorteil der Erfindung besteht in einer funktionellen Inaktivierung von Ribonukleinsäure-Molekülen von Transkriptionsfaktoren und Faktoren der Signaltransduktionswege zur Differenzierung und/oder Expression von Zytokinen, die an der Entstehung der chronischen Entzündungsreaktionen und Autoimmunerkrankungen beteiligt sind, mittels spezifischer DNAzyme und/oder siRNA. Diese Strategie zeichnet sich gegenüber konventionellen, aber auch gentherapeutischen Ansätzen durch höchste Zell- und/oder Gewebe- und/oder Krankheitsphasen-Spezifität und -Selektivität, hohe Stabilität der Moleküle und eine vernachlässigbare Antigenität aus. Es werden optimale Voraussetzungen für eine maßgeschneiderte Langzeittherapie bei Patienten mit chronischen Entzündungserkrankungen geschaffen.

Weitere Details und Vorzüge der vorliegenden Erfindung werden aus der folgenden Figur und der Beschreibung ersichtlich. Dabei zeigt
- **Fig. 1:**: schematische Darstellung der Signaltransduktion bei der Differenzierung von CD4⁺ Zellen zu TH1- bzw. TH2-Zell (modifiziert nach Ho I.C. und Glimcher L.H., Cell 2002; 109: S109-S120).
- **Fig. 2:**: Nukleotidsequenz der katalytischen Domäne des 10-23 DNAzym und Bindung an eine Ziel RNA mittels Watson-Crick Paarung. (R = A oder G; Y = U oder C, N = A, G, U oder G). Der Pfeil zeigt die Spaltstelle in der Ziel mRNA.
- **Fig. 3:**: Pool an spezifischen Ribonukleinsäure Molekülen nach Schritt b) im besonderen die DNAzyme hgd 1 bis hgd 70 gegen GATA-3 und ihre Nucleotidsequenzen (A=Adenin, G=Guanin, C=Cytosin, T=Thymin). Großgeschriebene Nukleotide markieren eine rechte und linke Substratbindungsdomäne, kleingeschriebene Nukleotide markieren die zentrale katalytische Domäne des 10-23 DNAzym.
- **Fig. 4:**: Nukleotidsequenzen humaner GATA-3-Gene im Alignment
Sequenz 1: Humanes GATA-3 aus Datenbank Nr.: XM_043124,
Sequenz 2: Humanes GATA-3 aus Datenbank Nr.: X58072.
Sequenz 3: Humanes GATA-3 (sequenziert aus Plasmid pCR2.1).
Divergente Basen sind grau unterlegt, Primerlokalisationen für die Klonierung von GATA-3 sind unterstrichen. Die Lokalisation des DNAzymes hgd40 ist mit fett geschriebenen Buchstaben, die gleichzeitig grau unterlegt und unterstrichen sind, verdeutlicht.
(A=Adenin, G=Guanin, C=Cytosin, T=Thymin)

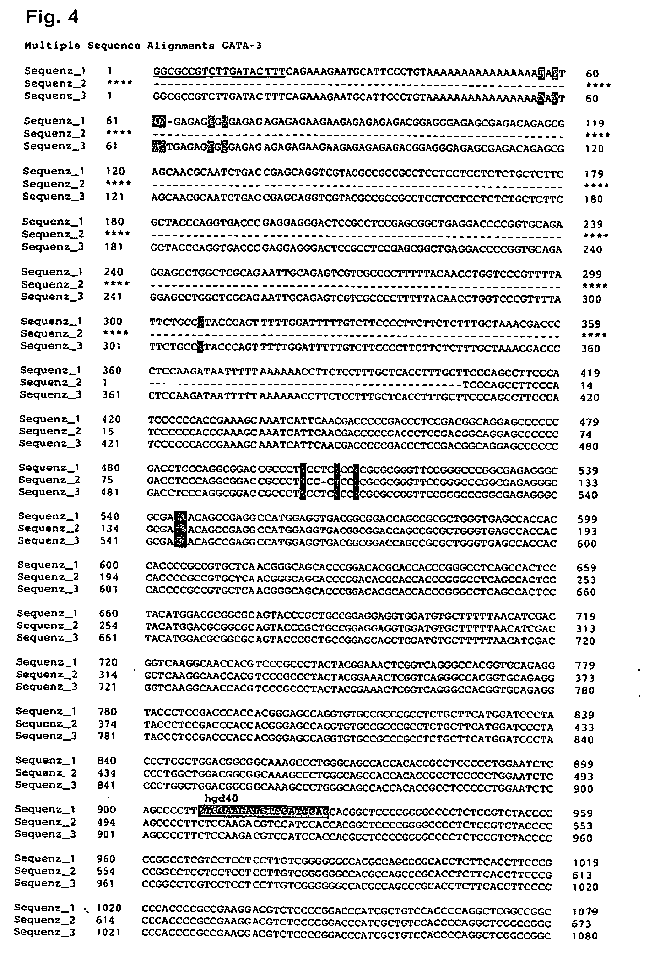

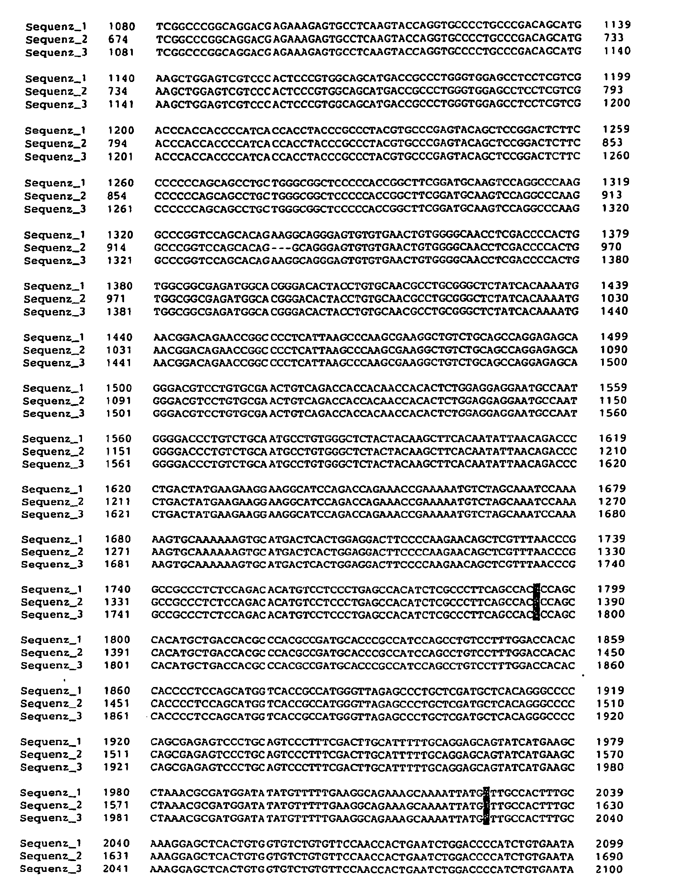

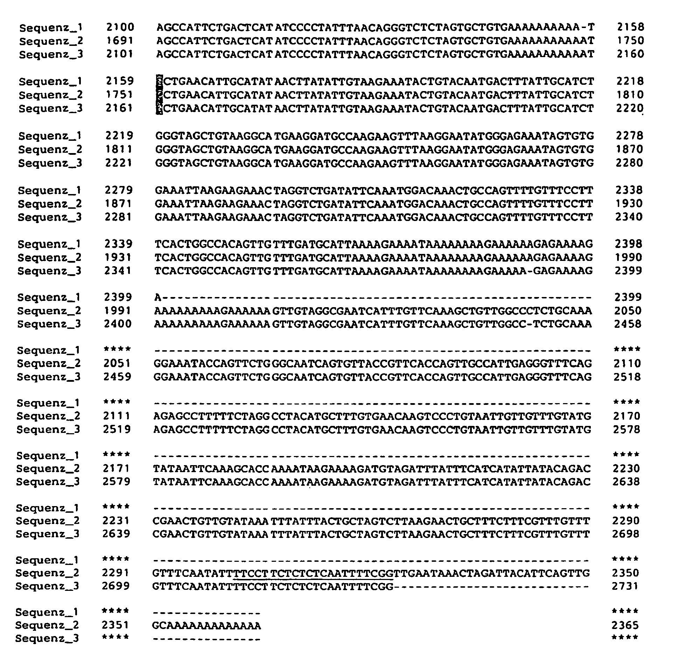

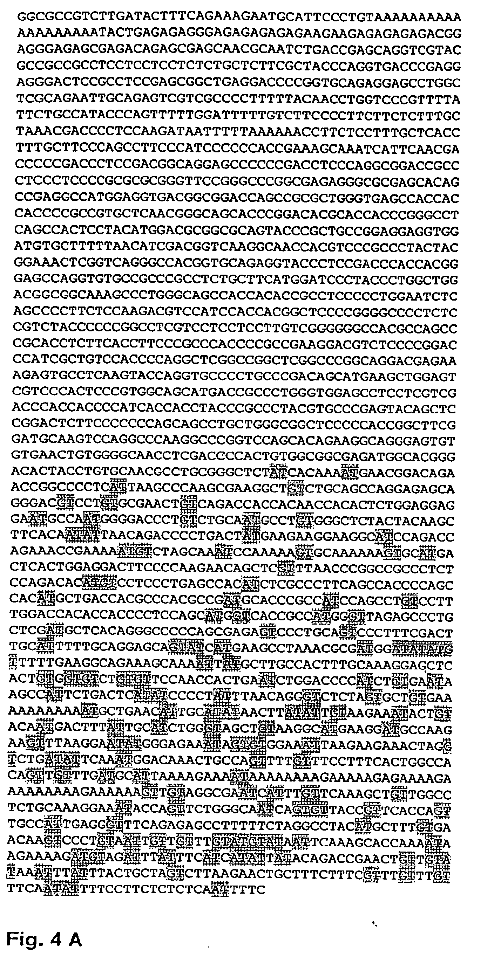

- **Fig. 4 A:**: Nukleotidsequenz 3 des humanen GATA-3-Gen aus Figur 4, darin eingezeichnet (grau hinterlegt) jeweils die Nukleotidpaare GT und AT, zwischen denen weitere DNAzyme-Schnittstellen liegen.

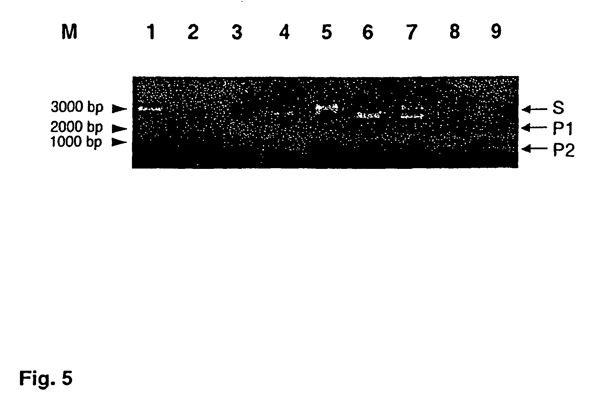

- **Fig 5:**: Gelelektrophorese zeigt die Spaltung einer Ziel-mRNA (hier GATA-3 mRNA) mit spezifischen Ribonukleinsäure Molekülen nach Schritt b), hier nicht modifizierte DNAzyme [hgd11 (Spur 2), hgd13 (Spur 4), hgd17 (Spur 6), hgd40 (Spur 8)] und modifizierten DNAzymen [hgd11-M (Spur 3), hgd13-M (Spur 5), hgd17-M (Spur 7), hgd40-M (Spur 9)]. M bezeichnet die modifizierten DNAzyme. Nicht modifizierte (0,25 µM) oder modifizierte DNAzyme (0,25 µM) werden eine Stunde bei 37°C mit in vitro transkribierter GATA-3 mRNA (0,025µM) in einem Volumen von 10 µl mit folgender Reaktionszusammensetzung inkubiert: 50 mM Tris pH 7,4, 150 mM NaCl, 10 mM MgCl₂. Anschließend werden die Produkte gelelektrophoretisch aufgetrennt. Spur 1 enthält als Kontrolle mRNA ohne DNAzyme-Zugabe. Mitgeführter Längenstandard (nicht dargestellt) zeigt Bandengrößen von 1000 bp, 2000 bp und 3000 bp. Pfeile zeigen auf S, die Bande mit dem Substrat (hier GATA-3-mRNA) und die Spaltprodukte P1 und P2.

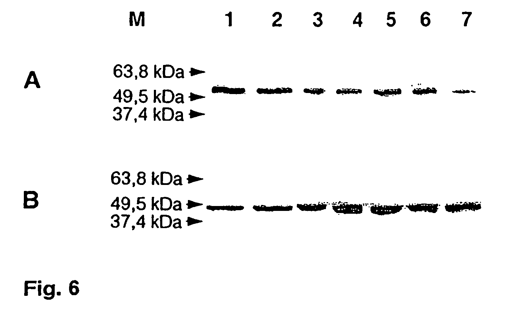

- **Fig. 6:**: Immunblot mit der Reaktion von spezifischen Ribonukleinsäure Molekülen in Zellen. Jurkat E6.1 Zellen werden mittels Lipofektion mit spezifischen Ribonukleinsäure Molekülen, hier DNAzyme [hgd11-M (Spur 4), hgd13-M (Spur 5), hgd17-M (Spur 6), hgd40-M (Spur 7)] transfiziert, Als Kontrollen werden nicht behandelte Zellen (Spur 1), nur mit Transfektionsmedium (Spur 2) behandelte Zellen, beziehungsweise mit DNAzymen (hgd11-M) ohne Transfektionsmedium (Spur 3) behandelte Zellen eingesetzt. Nach 48h Inkubation werden die solubilisierten Proteine mittels SDS-PAGE aufgetrennt und GATA-3 (A) durch Immunblot mit spezifischen Antikörpern detektiert. (Spur 4 enthält Zellen mit hgd11-M, Spur 5 enthält Zellen mit hgd13-M, Spur 6 enthält Zellen mit hgd17-M, Spur 7 enthält Zellen mit hgd40-M.) Zur Kontrolle auf gleiche Proteinmengen pro Spur wird auf der gleichen Blot-Membran eine Immunfärbung mit β-Aktin (B) durchgeführt. Mitgeführter Längenstandart (nicht dargestellt) zeigt Bandengrößen von 63,8 kDa, 49,5 kDa und 37,4 kDa.

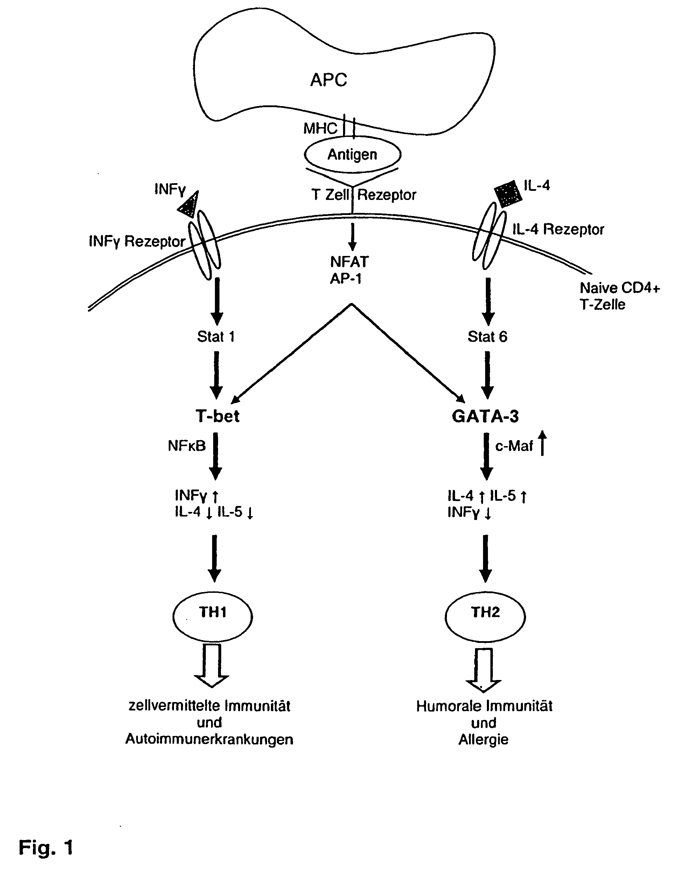

Figur 1 zeigt in einer nach Ho I.C. und Glimcher L.H. (Cell 2002; 109: S109- S120) modifizierten schematischen Darstellung die Zusammenhänge der Signaltransduktion bei der Differenzierung von CD4⁺ Zellen zu TH1- bzw. TH2-Zell. Die Stimulation über den T-Zellrezeptor durch den entsprechenden Peptid-MHC Komplex induziert die klonale Expansion und programmierte Differenzierung von CD4⁺ T-Lymphozten zu T-Helfer (TH)1- oder TH2-Zellen. Die Unterscheidung dieser beiden Subtypen erfolgt aufgrund ihrer Zytokin-Profile. TH1-Zellen produzieren Interferon-γ (INFγ), Interleukin 2 (IL-2) und Tumor-Nekrose-Faktor-β, wohingegen TH2-Zellen IL-4, IL-5, IL-9 und IL-13 sezernieren. Bakterielle und virale Infektionen induzieren eine Immunantwort, die von TH1-Zellen dominiert wird. Auf der anderen Seite regulieren TH2-Zellen die IgE Produktion gegen Parasiten. Dabei besteht zwischen TH1- und TH2-Zellen ein Gleichgewicht. Die Zerstörung dieses Gleichgewichtes verursacht Krankheiten, so ist eine überschießende TH1-Zellenantwort assoziiert mit Autoimmunerkrankungen, während allergischen Erkrankungen eine verstärkte TH2-Zellantwort zugrunde liegt.

Es ist bekannt, dass TH1-Zytokine in die Pathogenese von Autoimmunerkrankungen wie z.B. Autoimmunuveitis, experimentelle allergische Enzephalomyelitis, Typ 1 Diabetes mellitus oder Morbus Crohn involviert sind, während TH2-Zytokine (IL A, IL-5, IL-13 bzw. IL-9) an der Entstehung von chronisch entzündlichen Atemwegserkrankungen wie z.B. Atemwegseosinophilie, Mukus-Hypersekretion und Atemwegs-Hyperreagibilität beteiligt sind. Grundlage dieser Erkrankungen sind pathophysiologische Veränderungen während der Produktion von charakteristischen Zytokinen durch antigenspezifische TH-Zellen. So zeigen transgene Mäuse, die in den Atemwegsepithelien die TH2-Zytokine IL-4, IL-5, IL-13 bzw. IL-9 konstitutiv überexprimieren, typische allergische Entzündungsreaktionen. TH2-Zell-Subpopulationen in der Lunge und den Atemwegen rufen in TH2-Zellen im Tiermodell die charakteristischen Symptome des Asthma bronchiale hervor.

Überraschenderweise wurde gefunden, dass zur Zell- und/oder Gewebespezifisclien Behandlung von chronischen Entzündungen und/oder Autoimmunerkrankungen Transkriptionsfaktoren und Faktoren der Signaltransduktionswege zur Differenzierung und/oder Expression von Zytokinen, die an der Entstehung der chronischen Entzündungsreaktionen und Autoimmunerkrankungen beteiligt sind wie z.B.: der TH1-Zell-spezifische Transkriptionsfaktor T-bet und der TH2-Zellspezifische Transkriptionsfaktor GATA-3 in idealer Weise geeignet sind.

Der TH2-Zell-spezifische Transkriptionsfaktor GATA-3 ist vor allem für die Differenzierung von naiven CD4⁺ T-Zellen zu TH2-Zellen verantwortlich.

Die TH2-Zelldifferenzierung wird dabei hauptsächlich durch zwei Signalübertragungswege, den T-Zell Rezeptor- (TZR) und den IL-4 Rezeptor-Weg, gesteuert. Vom TZR weitergeleitete Signale aktivieren sowohl die TH2 zellspezitischen Transkriptionsfaktoren c-Maf und GATA-3 als auch die Transkriptionsfaktoren NFAT und AP-1. Die Aktivierung des IL-4 Rezeptors führt zur Bindung von STAT6 an die zytoplasmatische Domäne des IL-4 Rezeptors, wo er von Jak1- und Jak3-Kinasen phosphoryliert wird. Die Phosphorylierung ihrerseits führt zur Dimerisierung und Translokation von STAT6 in den Nukleus, wo STAT6 die Transkription von GATA-3 und anderen Genen aktiviert.
GATA-3 ist ein Zinkfinger-Transkriptionsfaktor, der nach "Representational-Difference-Analysis" (RDA) und Studien an transkriptioneller Regulation von IL-5 ausschließlich in maturen TH2-Zellen exprimiert wird, nicht in TH1-Zellen.
Weitere Transkriptionsfaktoren, die eine Rolle bei der Differenzierung zu TH1-beziehungsweise TH2-Zellen spielen und an der Entstehung der chronischen Entzündungsreaktionen und Autoimmunerkrankungen beteiligt sind weisen eine Expression auf, die sich in einer Zielzelle im Vergleich zur Expression einer Kontrollzelle unterscheidet und werden erfindungsgemäß ebenfalls zum Design von spezifischen DNAzymen und/oder siRNA für den therapeutischen Einsatz bei chronisch entzündlichen Erkrankungen eingesetzt:
- STAT4, STAT5a und STAT1 (signal transducer and activator of transcription)
- c-Rel
- CREB2 (cAMP response element-binding protein 2)
- ATF-2, ATF-2
- Hlx
- IRF-1 (interferon regulatory factor-1)
- c-Maf
- NFAT (Nuclear factor of activated T cells)
- NIP45 (NF-AT interacting protein 45)
- AP1 (Activator Protein 1)
- Mel-18
- SKAT-2 (SCAN box, KRAB domain associated with a Th2 phenotype)
- CTLA-4 (Cytolytic T lymphocyte-associated antigen 4)

Weitere Faktoren der Signaltransduktionswege, die zur Differenzierung und/oder Expression von Zytokinen verantwortlich sind und an der Entstehung der chronischen Entzündungsreaktionen und Autoimmunerkrankungen beteiligt sind, weisen eine Expression auf, die sich in einer Zielzelle im Vergleich zur Expression einer Kontrollzelle unterscheidet und werden erfindungsgemäß ebenfalls zum Design von spezifischen DNAzymen und/oder siRNA für den therapeutischen Einsatz bei chronisch entzündlichen Erkrankungen eingesetzt:
- Src kinase
- Tec kinase
   Rlk (Txk im Menschen)
   Itk
   Tec
- RIBP (Rlk/Itk-binding protein)
- PLCγ (Phospholipase Cγ1)
- MAP kinase (Mitogen-activated protein kinase)
   ERK
   JNK
   P38
- MKK (MAP kinase kinase)
   MKK1
   MKK2
   MKK3
   MKK4
   MKK6
   MKK7
- Rac2
- GADD45 (Growth arrest and DNA damage gene 45)
   GADD45β
   GADD45γ
- SOCS (Suppressors of cytokine signalling)
   CIS (Cytokine-induced SH2 protein)
   SOCS1
   SOCS2
   SOCS3
- JAK (Janus kinase)
   JAK1
   JAK3
- NIP45 (NF-AT interacting protein)

Erfindungsgemäß wird ein Zell- und/oder Gewebe- und/oder Krankheitsphasen spezifisches Arzneimittel bereitgestellt, das zur Behandlung von chronischen Entzündungen geeignet ist.
Das Arzneimittel greift bevorzugt an den Interventionspunkten der den chronischen Entzündungsreaktionen und Autoimmunerkrankungen zugrunde liegenden komplexen Kaskade der immunologischen und zellbiologischen Fehlregulationen an. Besonders bevorzugt sind dies Interventionspunkte der Regulation der Differenzierung der beteiligten Transkriptionsfaktoren, wie beispielsweise der TH2-Zell-spezifische Transkriptionsfaktor GATA-3. Der erzielte therapeutische Effekt besteht in einer funktionellen Inaktivierung von mRNA-Molekülen mittels spezifischer DNAzyme und/oder siRNA. Diese Strategie bietet eine Reihe von Vorteilen gegenüber konventionellen, aber auch gentherapeutischen Ansätzen: höchste Spezifität und Selektivität, hohe Stabilität der Moleküle und eine vernachlässigbare Antigenität. Es werden optimale Voraussetzungen geschaffen für eine maßgeschneiderte Langzeittherapie bei Patienten mit chronischen Entzündungserkrankungen.

Erfindungsgemäß wird ein Verfahren zur Herstellung eines Zell- und/oder Gewebe- und/oder Krankheitsphasen-spezifischen Arzneimittels bereitgestellt, dass folgende Schritte umfasst:
a) Identifikation von Ribonukleinsäure-Molekülen, deren Expression sich in einer Zielzelle im Vergleich zur Expression einer Kontrollzelle unterscheidet
b) Design von spezifischen Ribonukleinsäure-Molekülen, die an Ribonukleinsäure-Moleküle aus Schritt a) binden und sie funktionell inaktivieren
c) Einbringen der spezifischen Ribonukleinsäure-Moleküle aus Schritt b) in Zielzellen
d) Formulierung der spezifischen Ribonukleinsäure-Molekülen aus Schritt b) und/oder einer Zielzelle aus Schritt c) in einem Arzneimittel

Der Begriff "Zell- und/oder Gewebe- und/oder Krankheitsphasen-spezifisch" bedeutet im Sinne der vorliegenden Erfindung, dass das mittels des erfindungsgemäßen Verfahrens hergestellte Arzneimittel im wesentlichen nur bei einer bestimmten Art von Zellen (Zielzelle) und/oder in bestimmten Geweben oder Organen und/oder in bestimmten Phasen der Erkrankung wirksam ist, und einen vernachlässigbaren Einfluss auf andere Zellen (Kontrollzellen), Gewebe oder Organe besitzt. Vorzugsweise ist das Arzneimittel bei mindestens 2/3 der Zielzellen wirksam. Stärker bevorzugt bei mindestens 80% und am meisten bevorzugt bei mindestens 98% der Zielzellen. Es ist außerdem bevorzugt, dass das Arzneimittel bei höchstens 10% der Kontrollzellen wirksam ist, stärker bevorzugt bei höchstens 5% und am meisten bevorzugt bei < 1% der Kontrollzellen.

Der Begriff "Identifikation von Ribonukleinsäure-Molekülen, deren Expression sich in einer Zielzelle im Vergleich zur Expression einer Kontrollzelle unterscheidet" umfasst in der vorliegenden Erfindung folgende Punkte:
i) Zielzellen sind Zellen in Geweben und Organen, die bekannterweise zur Entstehung einer Krankheit führen, dazu beitragen oder diese verstärken, die die Krankheit aufrecht erhaltenden Prozesse unterhalten, zu ihnen beitragen oder dise verstärken, beziehungsweise die zu Spätfolgen einer Krankheit führen, beitragen oder sie verstärken. Dazu zählen beispielsweise Zellen, die bestimmte Transkriptionsfaktoren aufweisen, spezifische Hormone, Zytokine und Wachstumsfaktoren sezernieren, oder Zellen mit typischen Oberflächenrezeptoren.
ii) Die Zielzellen können zum Beispiel mittels Technologien isoliert werden, die auf der Bindung spezifischer Antikörper basieren. Hier werden Magnetic Beads, erhältlich von den Firmen Miltenyi (Macs-System), Dynal (DynaBeads) oder BD-Bioscience (iMAG) angewendet. Alternativ erfolgt dies über eine Zellreinigung mittels Fluoreszenz-markierter Antikörper an Zellsortern beispielsweise der Firma Cytomation (MOFLO) oder BD-Bioscience (FACS-Vantage). Die Reinheit der Zielzellen ist bevorzugt bei mindestens 80%, stärker bevorzugt bei mindestens 95% und am meisten bevorzugt bei mindestens 99%.
iii) Verfahren zur Isolierung der RNA sind z.B. in Sambrook and Russell, Molecular Cloning, A Laboratory Manual, 3. Auflage, Cold Spring Harbor Laboratory (2001), New York und Ausubel et al., Current Protocols in Molecular Biology, John Wiley & Sons (1998), New York, beschrieben. Außerdem ist es dem Durchschnittsfachmann möglich, kommerziell verfügbare Kits (Silika-Technologie) z.B. das RNeasy Kit von der Firma Qiagen, zur RNA Isolierung zu verwenden. Weiterhin ist es bevorzugt, direkt mRNA aus den Zielzellen durch Verwendung kommerzieller Kits beispielsweise der Firmen Qiagen (Oligotex mRNA Kit), Promega (PolyATract mRNA Isolation System) oder Miltenyi (mRNAdirect) zu reinigen.
iv) Die Identifizierung von mRNAs, die differentiell unterschiedlich sind, d.h. mRNAs, deren Expression in der Zielzelle gegenüber der Kontrollzelle erhöht ist, erfolgt beispielsweise mit kommerziell erworbenen Genchips (z.B. MWG, CLONTECH)] oder mit einem Filter-Hybridisierungsverfahren (z. Bsp. Unigene) nach Herstellerangaben. Alternativ werden differentielle mRNAs durch subtraktive Hybridisierung von cDNA, die zuvor aus der mRNA durch RT-Reaktion entstanden sind, hergestellt. Zu diesen dem Fachmann bekannten Verfahren, zählen beispielsweise die SSH-Methode (Firma Clontech) oder die RDA-Methode. Zu einer ebenfalls bevorzugten Anwendungsform gehört die Kombination von Chiptechnologie und subtraktiver Hybridisierung. Die Identifizierung der differenziell exprimierten Gene erfolgt unter Einsatz der Chiptechnologie mit Hilfe von kommerziell erhältlichen Programmen z.B. mit dem Vector Xpression Programm der Firma InforMax. Beim Einsatz von subtraktiver Hybridisierung erfolgt nach der Isolierung der differentiell exprimierten Gene anhand herkömmlicher, dem Fachmann geläufiger Verfahren wie Klonierung und anschließende Sequenzierung (siehe z.B. Sambrook and Russell, Molecular Cloning, A Laboratory Manual, 3. Auflage, Cold Spring Harbor Laboratory (2001), New York und Ausubel et al., Current Protocols in Molecular Biology, John Wiley & Sons (1998), New York) ein Sequenzabgleich in einer Datenbank wie z.B. Gene-Bank (www.ncbi.nlm.nih.gov).
   Die Expression in der Zielzelle unterscheidet sich im Vergleich zur Expression in einer Kontrollzelle. In einer Ausführungsform des erfindungsgemäßen Verfahrens ist die Expression in der Zielzelle im Vergleich zur Expression in einer Kontrollzelle erhöht, vorzugsweise mindestens um einen Faktor 1,5. In einer besonders bevorzugten Ausführungsform ist die Expression in der Zielzelle im Vergleich zur Expression in einer Kontrollzelle um mindestens einen Faktor 5 erhöht, und in einer am meisten bevorzugten Ausführungsform ist die Expression nur in der Zielzelle, jedoch nicht in der Kontrollzelle nachweisbar.

Der Begriff "Design von Ribonukleinsäure Molekülen, die an Ribonukleinsäure Moleküle aus Schritt a) binden und sie funktionell inaktivieren" umfasst im Sinne der vorliegenden Erfindung die Verwendung von RNA-inaktivierenden-DNA-Enzymen (DNAzymen) und/oder Small-Interfering-RNA (siRNA), die Ribonukleinsäure Moleküle funktionell inaktivieren.
Der Begriff DNAzyme umfasst dabei erfindungsgemäß DNA-Moleküle, die die ZielSequenz der Nukleinsäure, sowohl DNA als auch RNA, spezifisch erkennen und spalten.
Ein generelles DNAzyme-Modell stellt das "10-23"-Modell dar. DNAzyme des 10-23-Modells - auch als "10-23 DNAzyme" bezeichnet - besitzen eine katalytische 25 Domäne von 15 Desoxyribonukleinsäuren, welche von zwei Substratbindungsdomänen flankiert wird. Die Länge der Substratbindungsdomänen ist variabel, sie sind entweder gleich oder unterschiedlich lang. In einer bevorzugten Ausführung, beträgt die Länge der Substratbindungsdomänen zwischen 6 und 14 Nukleotide.
In einer besonders bevorzugten Ausführung sind die Substratbindungsdomänen vollständig komplementär zu der Region, die die Spaltstelle flankiert. Um die Ziel RNA zu binden und sie zu spalten, muss das DNAzyme jedoch..nicht unbedingt vollständig komplementär sein. In vitro Untersuchungen zeigen, dass DNAzyme des 10-23 Typs die Ziel mRNA an Purin-Pyrimidin Abfolge-Sequenzen spalten.

Um die DNAzyme in der Behandlung von Krankheiten zu verwenden, ist es bevorzugt, dass die DNAzyme so gut wie möglich gegen Degradation im Körper (im Blut, im intrazellulären Milieu usw.) stabilisiert sind. Eine bevorzugte Ausführung ist die Einführung einer 3'-3'-Inversion an einem oder mehreren Enden des DNA-zymes. Der Begriff 3'-3'-Inversion bezeichnet eine kovalente Phosphatbindung zwischen den 3'-Kohlenstoffen des terminalen Nukleotids und des angrenzenden Nukleotids. Dieser Typ von Bindung steht im Gegensatz zu der normalen Phosphatbindung zwischen den 3' und 5' Kohlenstoffen von aufeinander folgenden Nukleotiden. Dementsprechend wird bevorzugt, dass das Nukleotid am 3'-Ende der an das 3'-Ende der katalytischen Domäne angrenzenden Substratbindungsdomäne invers ist. Zusätzlich zu den Inversionen können die DNAzyme modifizierte Nukleotide oder Nukleotid-Verbindungen enthalten. Modifizierte Nukleotide beinhalten z.B. N3'-P5'-Phosphoramidat Verbindungen, 2'-O-Methyl-Substitutionen und Peptid-Nukleinsäure-Verbindungen. Ihre Herstellung ist dem Fachmann geläufig.

Obwohl die potentiellen DNAzyme-Schnittstellen ubiquitär vorkommen, sind diese oft durch die sekundäre Struktur der RNA blockiert und somit den DNAzymen unzugänglich. Daher werden aus einem Pool an DNAzymen diejenigen selektioniert, deren Schnittstellen frei zugänglich sind. Diese selektionierten DNAzyme sind aktiv, spalten die Ziel-mRNA und inaktivieren sie somit funktionell. Die Effizienz der Spaltung der mRNA durch die einzelnen DNAzyme wird entweder durch Einzeltestung jedes DNAzymes oder durch gekoppelte Testung mehrerer DNAzyme in "Multiplex-Assays" (beschrieben z. B. in Cairns et al., 1999) gezeigt.

Der Begriff siRNA umfasst erfindungsgemäß doppelsträngige, 21-23 Basen lange RNA-Moleküle, die zu einer spezifischen Degradation der komplementären Ziel mRNAs sowohl in vitro als auch in vivo führen. Es ist dem Fachmann anhand der Literatur (z.B. http://www.mpibpc.gwdg.de/abteilungen/100/105/index.html) bekannt, ausgehend von der Ziel-mRNA Sequenz siRNA-Moleküle herzustellen.

Die Wahrscheinlichkeit, dass sich unter drei ausgewählten siRNA-Molekülen mindestens ein hochaktives (Inhibition der Ziel-RNA um mindestens 80%) befindet wird in der Literatur mit mindestens 70% angegeben. Es werden aus einem Pool an siRNA-Molekülen diejenigen selektioniert, die zu einer spezifischen Degeneration der komplementären Ziel-mRNA sowohl in vitro als auch in vivo führen.

Der Begriff "Einbringen der spezifischen Ribonukleinsäure Moleküle aus Schritt b) in Zielzellen" umfasst im Sinne der vorliegenden Erfindung die Transfektion von Vektoren, insbesondere Plasmide, Cosmide, Viren oder Bakteriophagen, die die oben beschriebenen erfindungsgemäßen spezifischen Ribonukleinsäuremoleküle enthalten, in die Zielzellen. Vorzugsweise sind die Vektoren zur Transformation tierischer und humaner Zellen geeignet und erlauben die Integration der erfindungsgemäßen Ribonukleinsäuremoleküle. Verfahren zur Transfektion wie z.B. Lipofektion mittels DMRIE-C der Firma Invitrogen sind dem Fachmann aus der Literatur bekannt. Grundsätzlich sind dafür auch liposomale Vektoren geeignet. Die Zielmoleküle sind Transkriptionsfaktoren, Zellen, die Hormone, Zytokine und Wachstumsfaktoren sezernieren, aber auch Zellen, die auf der Oberfläche der exprimierte Rezeptoren tragen.
Als Kontrollzellen im Sinne der Erfindung werden gesunde Zellen des Zielgewebes, typgleiche Zellen aus anderen Kompartimenten desselben Patienten oder auch aus gesunden Individuen herangezogen.

Die Kultivierung der Zielzelle erfolgt in Nährmedien, die den Bedürfnissen der Zielzelle nach pH-Wert, Temperatur, Salzkonzentration, Antibiotika, Vitaminen, Spurenelementen und Belüftung entsprechend angepasst sind.
Der Begriff Patient bezieht sich gleichermaßen auf Menschen und Wirbeltiere. Damit kann das Arzneimittel in der Human- und Veterinärmedizin verwendet werden.

Der Begriff "Formulierung der spezifischen Ribonukleinsäure Moleküle aus Schritt b) oder einer Zielzelle aus Schritt c) in einem Arzneimittel" umfasst pharmazeutisch akzeptable Kompositionen, die Modifikationen und "Prodrugs" beinhalten, sofern sie nach zuverlässiger medizinischer Beurteilung keine übermäßige Toxizität, Irritationen oder allergische Reaktionen am Patienten auslösen. Der Terminus "Prodrug" bezieht sich auf Verbindungen, die zur Verbesserung der Aufnahme transformiert werden, wie beispielsweise durch Hydrolyse im Blut.

Bevorzugter weise ermöglicht die Formulierung, dass die spezifischen Ribonukleinsäure Moleküle den Patienten in Form einer pharmazeutisch akzeptablen Komposition entweder oral, rektal, parenteral, intravenös, intramuskulär oder subkutan, Intracisternal, intravaginal, intraperitoneal, intrathekal, intravasculär, lokal (Puder, Salbe oder Tropfen) oder in Sprayform verabreicht werden.

Dosierungsformen für die örtliche Administration des Arzneimittels dieser Erfindung schließen Salben, Puder, Sprays oder Inhalationsmittel ein. Die aktive Komponente wird unter sterilen Bedingungen mit einem physiologisch akzeptablen Trägerstoff und möglichen Preservativen, Puffern oder Treibmitteln,je nach Bedarf, vermischt.
Die Art der Dosierung wird vom behandelnden Arzt entsprechend den klinischen Faktoren bestimmt. Es ist dem Fachmann bekannt, dass die Art der Dosierung von verschiedenen Faktoren wie z.B. Körpergröße, Gewicht, Körperoberfläche, Alter, Geschlecht oder der allgemeinen Gesundheit des Patienten abhängig ist, aber auch von dem speziell zu verabreichenden Mittel, der Dauer und Art der Verabreichung und von anderen Medikamenten, die möglicherweise parallel verabreicht werden.

Das mit dem erfindungsgemäßen Verfahren hergestellte Arzneimittel weist eine hohe Patienten-, Krankheits-, Stadien- bzw. Phasenspezifität auf. Es bewirkt eine Zell-spezifische Intervention und ist spezifisch für Kompartimente und Organe. Es entstehen keine oder nur sehr geringe Reaktionen des Immunsystems gegen das Arzneimittel, und das Nebenwirkungsprofil steht in einem akzeptablen Verhältnis zu Schweregrad, Prognose und Verlauf der Erkrankung.
Das Arzneimittel kann zur Therapie gegen sämtliche Erkrankungsgruppen, die mit chronischen Entzündungen einhergehen, wie z.B. Autoimmunerkrankungen, Erkrankungen des rheumatischen Formenkreises (Manifestationen an u. a. Haut, Lunge, Niere, Gefäßsystem, Nervensystem, Bindegewebe, Bewegungsapparat, Endokrinem System), Allergische Soforttypreaktionen und Asthma, Chronisch-obstruktive Lungenerkrankungen (COPD), Arteriosklerose, Psoriasis und Kontaktekzem sowie gegen chronische Abstoßungsreaktionen nach Organ- und Knochenmarkstransplantation angewendet werden.

### Beispiel

### a) Identifikation von Ribonukleinsäure Molekülen, deren Expression in einer Zielzelle sich im Vergleich zur Expression einer Kontrollzelle unterscheidet

i) Als Zielzellen werden die für die Entstehung von chronischen Entzündungsreaktionen verantwortlichen naiven CD4⁺ Zellen verwendet.
ii) Die CD4⁺ Zielzellen werden über Magnetic Beads (Firma Miltenyi (Macs-System) Dynal (DynaBeads) oder BD-Bioscience (iMAG) isoliert, alternativ mittels Fluoreszenz-markierter Antikörper an Zellsortern beispielsweise der Firmen Cytomation (MOFLO) oder BD-Bioscience (FACS-Vantage).
iii) Isolierung der RNA erfolgt nach Standard-Methode, siehe Sambrook and Russell, Molecular Cloning, A Laboratory Manual, 3. Auflage, Cold Spring Harbor Laboratory (2001), New York und Ausubel et al., Current Protocols in Molecular Biology, John Wiley & Sons (1998), New York.
   Alternativ wird ein RNeasy Kit der Firma Qiagen verwendet, oder es erfolgt direkte Isolierung der mRNA aus CD4⁺Zielzellen mit Oligotex mRNA Kit der Firmen Qia-20 gen nach Herstellerangaben.
iv) Die Identifizierung von mRNAs, die differentiell unterschiedlich sind, d.h. mRNAs, deren Expression in der Zielzelle gegenüber der Kontrollzelle erhöht ist, erfolgt mittels Genchips (z.B. MWG, CLONTECH)], und die Identifizierung der differenziell exprimierten Gene mittels Vector Xpression Programm der Firma Infor-Max.
   Filter-Hybridisierungsverfahren (z. Bsp. Unigene) nach Herstellerangaben. Der Isolierung der differenziell exprimierten Gene schließt sich Klonierung, Sequenzierung (nach Standardvorschriften siehe z.B. Sambrook and Russell, Molecular Cloning, A Laboratory Manual, 3. Auflage, Cold Spring Harbor Laboratory (2001) und der Sequenzabgleich in der Gene-Datenbank (www.ncbi.nlm.nih.gov) an.
   Die Expression von GATA-3 unterscheidet sich in der Zielzelle (TH2-Zelle) im Vergleich zur Expression in einer Kontrollzelle (beispielsweise Th0-Zelle).

### b) Design von spezifischen 3übcnukleinsäure Molekülen, die an Ribonukleinsäure Moleküle aus Schritt a) bänden und sie funktionell inaktivieren

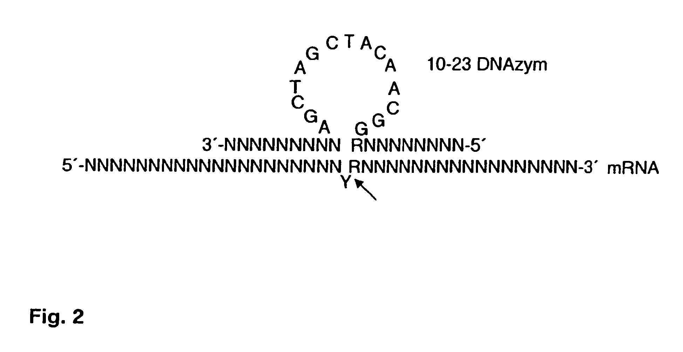

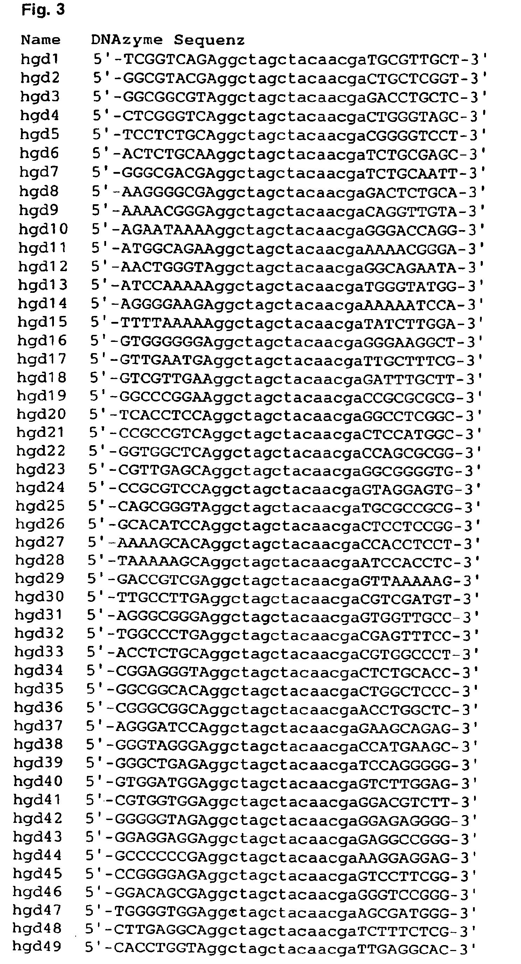

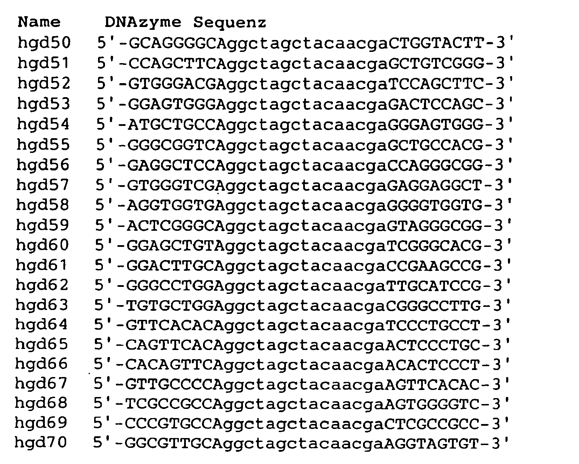

Figur 3 zeigt den erfindungsgemäßen Pool hgd1 bis hgd70 an spezifischen DNAzymen gegen GATA-3 mRNA. Die DNAzyme weisen eine Gesamtlänge von 33 Nukleotiden auf, wobei die zentrale katalytische Domäne 15 Nukleotiden (in kleingeschriebenen Buchstaben) der katalytischen Domäne des bekannten 10-23 DNAzyme (Figur 2) entspricht. Diese katalytische Domäne wird von zwei aus jeweils 9 Nukleotiden bestehenden rechten und linken Substratbindungsdomänen (in großgeschriebenen Buchstaben) flankiert. Die Nukleotidsequenz der rechten und linken Substratbindungsdomäne ist unterschiedlich und variiert bei den DNAzymen hgd1 bis hgd70, so dass eine unterschiedlich spezifische Bindung mittels Watson-Crick Paarung an die GATA-3 mRNA erfolgt.

Figur 2 zeigt das allgemeine Modell zur Bindung des 10-23 DNAzyme an eine mit N markierte beliebige Ziel RNA, wobei der Pfeil auf die Spaltstelle in der Ziel mRNA hinweist.
DNAzyme können die Ziel-mRNA zwar an jeder Purin-Pyrimidin Sequenz spalten, aus der Literatur ist jedoch bekannt, dass Purin-Uracil-Bindungen effektiver gespalten werden als Purin-Cytosin-Bindungen. Deshalb werden vorzugsweise DNAzyme konstruiert, die an Purin-Uracil-Bindungen spalten.
Das in Figur 2 gezeigte Modell kann in seiner Funktionsweise auf die Bindung der DNAzyme hgd1 bis hgd70 an GATA-3 mRNA übertragen werden.

Die DNAzyme hgd1 bis hgd70 werden für in vitro Versuche unmodifiziert eingesetzt, für Versuche in Zellkultur mit Modifikationen versehen (käuflich erworben durch Firma Eurogentec).
Als Modifikationen zur Stabilisierung und Schutz werden eingesetzt:
1) Ein stabilisierendes inverses Thymidin am 3'-Ende
2) eine FAM-Markierung am 5'-Ende zur Beurteilung der Transfektionseffizienz der Zellen mittels FACS-Analyse.
Um die DNAzyme in vitro zu testen, wird GATA-3 mRNA benötigt, die durch in vitro Transkription hergestellt wird. Die einzelnen Schritte sind wie folgt:
- RNA-Isolierung aus humanem EDTA-Vollblut mittels QlAamp-RNA-Blood-Mini-Kit (Qiagen, Deutschland) nach Herstellerangaben
- reverser Transkription mit den Primern:
   Forward-Primer GGCGCCGTCTTGATACTTT
   Revers-Primer CCGAAAATTGAGAGAGAAGGAA, wobei ein PCR-Produkt mit einer
   Länge von 2731 Nukleotiden amplifiziert wird (JumpStart Accu Taq DNA Polymerase, Sigma).
PCR Bedingungen: Initiale Denaturierung (96°C, 30 Sek.) Amplifikation mit 40 Zyklen (94°C, 15 Sek.; 48°C, 30 Sek.; 68°C, 3 Min.), Final Extention (68°C, 30 Min).
Das PCR-Produkt wird mittels Standardverfahren in das Plasmid pCR2.1 (Invitrogen) kloniert und zur Überprüfung sequenziert. Die Herstellung von GATA-3 mRNA erfolgt nach Linearisierung des GATA-3 enthaltenden Plasmids pCR2.1 durch Spaltung mit dem Restriktionsenzym Spe 1 durch in vitro Transkription nach Herstellerangaben (Ambion). GATA-3 mRNA liegt mit einer Länge von insgesamt 2876 Nukleotiden vor.

Figur 4 zeigt die bekannten Nukleotidsequenzen humaner GATA-3-Gene aus Datenbankeinträgen [PubMed (http://www.ncbi.nlm.nih.gov/entrez/query. fcgi?db=Nucleotide)], wobei divergente Basen grau unterlegt sind. Sequenz 1: Humanes GATA-3 aus Datenbank Nr.: XM_043124, Sequenz 2: Humanes GATA-3 aus Datenbank Nr.: X58072, Sequenz 3: Humanes GATA-3 (isoliert aus Plasmid pCR2.1).
Die GATA-3 mRNA Sequenzen unterscheiden sich bezüglich der Länge der 3'-untranslatierten oder 5'- untranslatierten Enden voneinander. Um die exakte Gesamt-Sequenz der mRNA zu erhalten, werden zur Primerselektion die mRNA Sequenzen der Einträge Nr.: XM_043124 und X58072 verwendet. Die Primerlokali sationen für die Klonierung von GATA-3 sind in Figur 4 als Unterstreichung hervorgehoben. Figur 4 zeigt weiterhin ein Alignment der Nukleinsäuresequenz von GATA-3 aus der Datenbank (Sequenz 1 und 2) mit der aus Plasmid pCR2.1 sequenzierten Nukleotidsequenz (Sequenz 3). Dabei zeigt sich, dass die Sequenzen nicht vollkommen identisch, sondern einzelne Basen unterschiedlich sind. Die Nukleinsäuresequenz 3 von GATA-3 aus Figur 4 bildet erfindungsgemäß die Grundlage für die Konstruktion von DNAzymen gegen GATA-3 mRNA.

Figur 4A zeigt die Nukleotidsequenz der Sequenz 3 des humanen GATA-3-Gen aus Figur 4 und darin als graue Hinterlegung eingezeichnet jeweils zwei Nukleotide GT bzw. AT, zwischen denen weitere potentielle Schnittstellen für DNAzyme liegen.

Die in vitro Spaltungsexperimente von GATA-3 mRNA mit den DNAzymen (hgd1-hgd70) werden in einem Volumen von 10 µl folgender Reaktionszusammensetzung durchgeführt: 50 mM Tris pH 7,4, 150 mM NaCl, 10 mM MgCl2, 0,25 µM DNAzyme und 0,025 µM in vitro transkribierte GATA-3 mRNA (in einem Substrat zu DNAzyme Verhältnis von 1:10). Die Reaktionen werden bei 37°C für die jeweils angegebenen Zeiten inkubiert. Durch die Zugabe von Formamid- und EDTA-haltigem RNA-Sample-Loading-Buffer (Sigma) wird die Reaktion gestoppt. Die denaturierten Proben werden in 1,3 %igen TAE-Agarose-Gelen aufgetrennt und im UV-Transilluminator analysiert. Figur 5 zeigt als Ergebnis der Gelelektrophorese die Spaltung der GATA-3 Ziel mRNA mit nicht modifiziertem DNAzyme [hgd11 (Spur 2), hgd13 (Spur 4), hgd17 (Spur 6), hgd40 (Spur 8)] und modifizierten DNAzymen [hgd11-M (Spur 3), hgd13-M (Spur 5), hgd17-M (Spur 7), hgd40-M (Spur 9)]. Spur 1 enthält als Kontrolle mRNA ohne DNAzym-Zugabe. Die modifizierten DNAzyme sind mit einem zusätzlichen M gekennzeichnet. Ein mitgeführter Längenstandard (nicht dargestellt) zeigt Bandengrößen von 1000 bp, 2000 bp und 3000 bp. Pfeile zeigen auf S, die Bande mit dem Substrat (hier GATA-3-mRNA) und die Spaltprodukte P1 und P2.

Der Vergleich zwischen allen 70 DNAzymen zeigt, dass hgd11, hgd13, hgd17 und hgd40 besonders aktiv sind, die Modifikationen die Effektivität der DNAzyme hgd11, hgd13 und hgd17 herabsetzt, nicht jedoch die Effektivität des DNAzymes hgd40.
Die folgende Tabelle zeigt die Einteilung der DNAzyme hgd 1 bis hgd 70 gegen GATA-3 mRNA in 4 Gruppen. Diese Gruppeneinteilung erfolgt aufgrund durchgeführter in-vitro Aktivitätstestungen der DNAzyme gegen GATA-3 mRNA. Gruppe 1: hohe Spaltungsaktivität, Gruppe 2: mittlere Spaltungsaktivität, Gruppe 3: schwache Spaltungsaktivität und Gruppe 4: keine messbare Spaltungsaktivität.

| **Gruppe** | **hgd** | **Aktivität gegen GATA-3 mRNA** |
|---|---|---|
| 1 | 11, 13,17, 40 | Hohe Spaltungsaktivität |
| 2 | 10, 12, 16, 18, 23, 31, 36, 37, 39, 52, 57, 58, 63, 70 | Mittlere Spaltungsaktivität |
| 3 | 22, 24, 25, 34, 35, 41, 42, 43, 45, 46, 47, 48, 49, 50, 54, 55, 56, 57 | Schwache Spaltungsaktivität |
| 4 | 1, 2, 3, 4, 5, 6, 8, 9, 14, 15, 19, 20, 21, 26, 27, 28, 29, 30, 31, 32, 33, 38, 44, 51, 53, 59, 60, 61, 62, 64, 65, 66, 68, 69 | Keine Spaltungsaktivität |

### c) Einbringen der spezifischen Ribonukleinsäure Moleküle aus Schritt b) in Zielzellen

Die hoch aktiven DNAzyme hgd11, hgd13, hgd17 und hgd40 werden mit und ohne die beschriebenen Modifikationen in Zielzellen verwendet.

Dazu werden Jurkat E6.1 Zellen (human acute T cell leukemia Cells) im RPMI-Medium mit 100 U/ml Penicillin, 0,1 mg/ml Streptomycin und 10% FKS bei 37°C in befeuchteter 5 %iger CO2-Atmosphäre kultiviert. Die Transfektionen werden in 6-Wellplatten durchgeführt. Hierfür werden 2x10⁶ Jurkat E6.1 Zellen in Opti-MEM-I-Zellkulturmedium (Invitrogen) überführt und mittels DMRIE-C (Invitrogen) mit den modifizierten DNAzymen (0,3 µM) transfiziert (nach Herstellerangaben der Firma Invitrogen). Nach 10 Stunden Inkubation im Brutschrank unter obigen Bedingungen wird RPMI-Medium (mit den oben angegebenen Zusätzen) hinzu gegeben und die Inkubation für weitere 14 Stunden fortgesetzt. Die Zellen werden mit Opti-MEM-Medium gewaschen und anschließend erneut nach dem oben beschriebenen Protokoll transfiziert. Nach jeder Transfektion wird die Transfektionseffizienz mittels FACS-Analyse beurteilt.

Anschließend wird die Aktivität der DNAzyme durch Nachweis der GATA-3 Proteinmenge im Westernblot überprüft (siehe Figur 6).

Für Westernblot-Analysen werden die zytoplasmatischen Proteine und die Kern proteine mittels Protein-Extraction-Kit nach Herstellerangaben (Pierce) getrennt. aufgearbeitet. Die Proteinkonzentration wird mit dem BCA-Kit (Pierce) bestimmt. Die Auftrennung von jeweils 30 µg Protein erfolgt mittels denaturierender GelElektrophorese in 10 %igen SDS-Polyacrylamide-Gelen. Die Proteine werden anschließend nach Standardverfahren auf Nitrocellulose-Membranen geblottet. Die Membranen werden mit 5 % Magermilchpulver in PBS (mit 0,01 % Tween 20) geblockt und anschließend mit Maus-Anti-GATA-3 Antikörpern (Santa Cruz) (1:500) und darauf folgend mit HRP-gekoppelten Maus-Anti-Kaninchen Antikörpern (BD Biosciences) (1:2000) für jeweils eine Stunde bei Raumtemperatur inkubiert. Die Proteine werden mittels Chemilumineszenz visualisiert. Durch den parallelen Nachweis von Beta-Aktin auf den Blots werden Variationen in der Protein-Auftragsmenge kontrolliert. Dafür wird auf der Nitrozellulose-Membran zuerst GATA-3 detektiert. Anschließend wird dieselbe Membran über Nacht in einer feuchten Kammer belassen. Nach 2 maligem Waschen mit PBS erfolgt der Nachweis von 13-Aktin durch Immunfärbung mit spezifischen Antikörpern (Maus-anti-Human Beta-Aktin Antikörper (Sigma)).
Figur 6 zeigt das Resultat des Immunblot mit dem Ergebnis der Aktivität der DNAzyme in Zellen. Jurkat E6.1 Zellen werden mittels Lipofektion mit DNAzymen (Spur 4=hgd11-M, Spur 5=hgd13-M, Spur 6=hgd17-M, Spur 7=hgd40-M) transfiziert. Als Kontrollen werden nicht behandelte (Spur 1), nur mit Transfektionsmedium (Spur 2), beziehungsweise mit DNAzymen ohne Transfektionsmedium (Spur 3) behandelte Zellen eingesetzt. Nach 48h Inkubation werden die solubilisierten Proteine mittels SDS-PAGE aufgetrennt und GATA-3 (A) durch Immunblot mit spezifischen Antikörpern detektiert. Um zu bestätigen, dass in jeder Spur die gleiche Menge an Protein eingesetzt wird, erfolgt auf der gleichen Blot-Membran eine Immunfärbung mit β-Aktin (B). Ein mitgeführter Längenstandard (nicht dargestellt) zeigt Proteinbanden der Größe 63,8 kDa, 49,5 kDa und 37,4 kDa.

Es zeigt sich, dass die DNAzyme hgd11, hgd13 und hgd17 in vivo nicht aktiv sind, wohingegen das DNAzyme hgd40 die GATA-3 Expression auch in vivo inhibiert, Die spezifische Inhibition der GATA-3 Expression in vivo durch das DNAzyme hgd40 stellt somit ein effektives therapeutisches Werkzeug zur Behandlung von chronisch entzündlichen Erkrankungen dar.

### d) Formulierung der spezifischen Ribonukleinsäure aus Schritt b) und/oder einer Zielzelle aus Schritt c) in einem Arzneimittel

Die Analyse verschiedener DNAzyme mit für GATA-3 spezifischer Substratbindedomäne zeigt, dass DNAzyme hgd40 die GATA-3 Expression in vivo spezifisch inhibiert und als spezifische Ribonukleinsäure zur Herstellung eines Zell- und/oder Gewebe- und/oder Krankheitsphasen-spezifischen Arzneimittels geeignet ist. Dazu wird hgd40 (5'-GTGGATGGAggctagctacaacgaGTCTTGGAG) oder mit hgd40 transfizierte Zellen in einer pharmazeutischen Komposition mit einem pharmazeutisch akzeptablen Carrier beispielsweise Liposome oder bioabbaubare Polymere versehen.
Alternativ zu den DNAzymen wird zur spezifischen Inhibition der GATA-3 Expression und zur Herstellung eines Zell- und/oder Gewebe- und/oder Krankheitsphasen-spezifischen Arzneimittels der Einsatz von zu siRNA vorgeschlagen. Vorzugsweise wird siRNA zur Inhibition von Maus und humanem GATA-3 eingesetzt. Die Herstellung der siRNA ist dem Fachmann bekannt und in der Literatur beschrieben.
Beispiele für siRNA Sequenzen sind unten aufgeführt:

| Quelle | Nukleinsäuresequenzen |
|---|---|
| Maus GATA-3 | Sense-Strang: CAUCGAUGGUCAAGGCAACdTdT Antisense-Strang: GUUGCCUUGACCAUCGAUGdTdT |
| Humane GATA-3 Sequenz 1 | Sense-Strang: CAUCGACGGUCAAGGCAACdTdT Antisense-Strang: GUUGCCUUGACCGUCGAUGdTdT |
| Humane GATA-3 Sequenz 2 | Sense-Strang: AAGAGUGCCUCAAGUACCAdTdT Antisense-Strang: UGGUACUUGAGGCACUCUUdTdT |
| Humane GATA-3 Sequenz 3 | Sense-Strang: AGCUUCACAAUAUUAACAGdTdT Antisense-Strang: CUGUUAAUAUUGUGAAGCUdTdT |
| Humane GATA-3 Sequenz 4 | Sense-Strang: UGACUCACUGGAGGACUUCdTdT Antisense-Strang: GAAGUCCUCCAGUGAGUCAdTdT |

Dem Fachmann ist ersichtlich, dass mit dem Wissen der vorliegenden Erfindung auch leicht spezifische DNAzyme bzw. siRNAs als Arzneimittel bei chronisch entzündlichen Erkrankungen und Autoimmunerkrankungen herstellbar sind, die gegen weitere Transkriptionsfaktoren gerichtet sind, die eine Rolle bei der Differenzierung zu TH1- beziehungsweise TH2-Zellen spielen beispielsweise STAT4, STAT5a, STAT1, c-Rel, CREB2, ATF-2, ATF-2, Hlx, IRF-1, c-Maf, NFAT, NIP45, AP1, Mel-18, SKAT-2, CTLA-4 oder die gegen weitere Faktoren der Signaltransduktionswege zur Differenzierung und/oder Expression von Zytokinen gerichtet sind, beispielsweise Src kinase, Tec kinase, Rlk (Txk im Menschen), Itk, Tec, RIBP, PLCγ, MAP kinase, ERK, JNK, P38, MKK, MKK1, MKK2, MKK3, MKK4, MKK6, MKK7, Rac2, GADD45, GADD45β, GADD45γ, SOCS, CIS, SOCS1, SOCS2, SOCS3, JAK, JAK1, JAK3, NIP45.

Diese Proteine weisen eine Expression auf, die in einer Zielzelle im Vergleich zur Expression einer Kontrollzelle erhöht ist

## Patentansprüche

1. DNAzyme, **dadurch gekennzeichnet, dass** sie bestehen aus
- einer katalytischen Domäne mit der Nukleotidsequenz GGCTAGCTACAACGA, oder einer modifizierten Sequenz mit vergleichbarer biologischer Wirkung, die die GATA-3 mRNA an jeder Purin-Pyrimidin Bindungsstelle schneidet, an der sie gebunden ist,
- einer rechten Substratbindedomäne, die sich am 3'-Ende der katalytischen Domäne anschließt und
- einer linken Substratbindedomäne, die sich am 5'-Ende der katalytischen Domäne anschließt, wobei die beiden Substratbindedomänen jeweils komplementär zu zwei Regionen der GATA-3 mRNA sind, so dass sie mit der mRNA hybridisieren,
- in vivo aktiv sind und
- die Sequenz hgd 40 GTGGATGGA GGCTAGCTACAACGA GTCTTGGAG enthalten.

2. DNAzyme nach Anspruch 1, **dadurch gekennzeichnet, dass** sie mit der katalytischen Domäne die GATA-3 mRNA an einer Purin-Uracil Bindungsstelle schneiden.

3. DNAzyme nach den Ansprüchen 1 und 2, **dadurch gekennzeichnet, dass** sie gegen den Abbau im Organismus durch die Einführung einer 3'-3' Inversion stabilisiert sind.

4. DNAzyme nach den Ansprüchen 1 bis 3, **dadurch gekennzeichnet, dass** sie gegen den Abbau im Organismus durch die Einführung von modifizierten Nukleotiden oder Nukleotidverbindungen stabilisiert sind.

5. DNAzyme nach den Ansprüchen 1 bis 4, **dadurch gekennzeichnet, dass**, sie als Modifikation ein inverses Thymidin am 3'-Ende und/oder eine FAM-Markierung am 5'-Ende aufweisen.

6. Verfahren zur Herstellung eines Arzneimittels zur Behandlung von chronischen Entzündungen, **dadurch gekennzeichnet, dass** man
- Zielzellen ermittelt, in denen die Expression von GATA-3 mRNA höher ist als in gesunden Körperzellen,
- ein vivo wirksames DNAzym gemäß Anspruch 1 entwickelt, das an GATA-3 mRNA bindet und sie funktionell inaktiviert,
- das in vivo wirksame DNAzym in die Zielzellen einbringt und
- Arzneimittel formuliert, die das in vivo wirksame DNAzym enthalten.

7. Arzneimittel enthaltend ein DNAzym gemäß den Ansprüchen 1 bis 5 und einen pharmazeutisch akzeptablen Carrier.

8. Verwendung eines DNAzyms gemäß einem oder mehreren der Ansprüche 1 bis 5 zur Herstellung eines Arzneimittels zur Behandlung von chronischen Entzündungen.

9. Verwendung eines DNAzyms gemäß einem oder mehreren der Ansprüche 1 bis 5 zur Herstellung eines Arzneimittels zur spezifischen Inhibition der GATA-3 Expression in Zielzellen von Patienten, welche an chronischen Entzündungen leiden.

## Claims

1. DNAzymes **characterised in that** they consist of
- a catalytic domain with the nucleotide sequence GGCTAGCTACAACGA or a modified sequence with a comparable biological effect which intersects GATA-3 mRNA at each purine-pyrimidine binding site, to which it is bound,
- a right hand substrate binding domain which adjoins at the 3' end of the catalytic domain and
- a left hand substrate binding domain which adjoins at the 5' end of the catalytic domain, the two substrate binding domains being complementary to two regions of GATA-3 mRNA such that they hybridise with mRNA
- are active in vivo and
- contain the sequence hgd 40 GTGGATGGA GGCTAGCTACAACGA GTCTTGGAG.

2. DNAzymes according to claim 1 **characterised in that** they intersect, with the catalytic domain, GATA-3 mRNA at a purine-uracil binding site.

3. DNAzymes according to claims 1 and 2 **characterised in that** they are stabilised against degradation in the organism by the introduction of a 3'-3' inversion.

4. DNAzymes according to claims 1 to 3 **characterised in that** they are stabilised against degradation in the organism by the introduction of modified nucleotides or nucleotide compounds.

5. DNAzymes according to claims 1 to 4 **characterised in that** they exhibit, as modification, an inverse thymidine at the 3' end and/or a FAM label at the 5' end.

6. Process for the production of a drug for the treatment of chronic inflammations **characterised in that**
- target cells are determined in which the expression of GATA-3 mRNA is higher than in healthy body cells,
- a DNAzyme effective in vivo according to claim 1 is developed which binds to GATA-3 mRNA and inactivates it functionally,
- the DNAzyme effective in vivo is introduced into the target cells and
- drugs are formulated which contain the DNAzyme active in vivo.

7. Drug containing a DNAzyme according to claims 1 to 5 and a pharmaceutically acceptable carrier.

8. Use of a DNAzyme according to one or several of claims 1 to 5 for the production of a drug for the treatment of chronic inflammations.

9. Use of a DNAzyme according to one or several of claims 1 to 5 for the production of a drug for the specific inhibition of the T-bet expression in target cells of patients for the treatment of chronic inflammations.

## Revendications

1. Désoxyribozymes, **caractérisées en ce qu'**elles sont constituées
- d'un domaine catalytique ayant la séquence de nucléotides GGCTAGCTACAACGA, ou d'une séquence modifiée exerçant un effet biologique comparable, coupant l'ARNm GATA-3 à chaque liaison purine-pyrimidine à laquelle il est lié,
- d'un domaine de liaison au substrat qui prolonge le domaine catalytique sur le côté droit, c'est-à-dire à l'extrémité 3', et
- d'un domaine de liaison au substrat qui prolonge le domaine catalytique sur le côté gauche, c'est-à-dire à l'extrémité 5', les deux domaines de liaison au substrat étant chacun complémentaire à deux régions de l'ARNm GATA-3, faisant en sorte qu'ils s'hybrident avec l'ARNm,
- sont actives in vivo, et
- contiennent la séquence hgd 40 GTGGATGGA GGCTAGCTACAACGA GTCTTGGAG.

2. Désoxyribozymes selon la revendication 1, **caractérisées en ce qu'**elles coupent, à l'aide du domaine catalytique, l'ARNm GATA-3 à une liaison purine-uracile.

3. Désoxyribozymes selon les revendications 1 et 2, **caractérisées en ce qu'**elles sont stabilisées contre une dégradation dans l'organisme par l'introduction d'une inversion 3'-3'.

4. Désoxyribozymes selon les revendications 1 et 3, **caractérisées en ce qu'**elles sont stabilisées contre une dégradation dans l'organisme par l'introduction de nucléotides ou de composés de nucléotides modifiés.

5. Désoxyribozymes selon les revendications 1 à 4, **caractérisées en ce qu'**elles ont été modifiées de façon à ce qu'elles présentent une thymidine inversée à l'extrémité 3' et/ou un marqueur FAM à l'extrémité 5'.

6. Procédé de préparation d'un médicament destiné au traitement d'inflammations chroniques, **caractérisé en ce qu'**on
- détermine les cellules cibles dans lesquelles le niveau d'expression d'ARNm GATA-3 est plus élevé que dans des cellules saines du corps,
- développe une désoxyribozyme selon la revendication 1 qui est dotée d'une efficacité in vivo et qui entre en liaison avec l'ARNm GATA-3 pour inactiver les fonctions de ce dernier,
- introduit dans les cellules cibles la désoxyribozyme dotée d'une efficacité in vivo, et
- formule des médicaments contenant la désoxyribozyme dotée d'une efficacité in vivo.

7. Médicament contenant une désoxyribozyme selon les revendications 1 à 5 ainsi qu'un support pharmaceutiquement acceptable.

8. Utilisation d'une désoxyribozyme selon une ou plusieurs des revendications 1 à 5 pour préparer un médicament destiné au traitement d'inflammations chroniques.

9. Utilisation d'une désoxyribozyme selon une ou plusieurs des revendications 1 à 5 pour préparer un médicament destiné à l'inhibition spécifique de l'expression de T-bet dans les cellules cibles de patients pour ainsi traiter des inflammations chroniques.


### SEQUENCE LISTING

<110> Philipps-universität Marburg
<120> verfahren zur Herstellung eines zell- und/oder Gewebe- und/oder Krankheitsphasen-spezifischen Arzneimittels
<130> 04802618.1 - 2403
<140> PCT/DE2004002197
   <141> 2004-10-01
<150> DE 10346487.5
   <151> 2003-10-02
<160> 73
<170> PatentIn version 3.3
<210> 1
   <211> 33
   <212> DNA
   <213> Homo sapiens
<220>
   <221> hgd1 DNAzyme against GATA-3mRNA
   <222> (1)..(33)
<400> 1
   tcggtcagag gctagctaca acgatgcgtt gct 33
<210> 2
   <211> 33
   <212> DNA
   <213> Homo sapiens
<220>
   <221> hdg2 DNAzyme against GATA-3mRNA
   <222> (1)..(33)
<400> 2
   ggcgtacgag gctagctaca acgactgctc ggt 33
<210> 3
   <211> 33
   <212> DNA
   <213> Homo sapiens
<220>
   <221> hgd3 DNAzyme against GATA-3mRNA
   <222> (1)..(33)
<400> 3
   ggcggcgtag gctagctaca acgagacctg ctc 33
<210> 4
   <211> 33
   <212> DNA
   <213> Homo sapiens
<220>
<221> hgd4 DNAzyme against GATA-3mRNA
   <222> (1)..(33)
<400> 4
   ctcgggtcag gctagctaca acgactgggt agc 33
<210> 5
   <211> 33
   <212> DNA
   <213> Homo sapiens
<220>
   <221> hgd5 DNAzyme against GATA-3mRNA
   <222> (1)..(33)
<400> 5
   tcctctgcag gctagctaca acgacggggt cct 33
<210> 6
   <211> 33
   <212> DNA
   <213> Homo sapiens
<220>
   <221> hgd6 DNAzyme against GATA-3mRNA
   <222> (1)..(33)
<400> 6
   actctgcaag gctagctaca acgatctgcg agc 33
<210> 7
   <211> 33
   <212> DNA
   <213> Homo sapiens
<220>
   <221> hgd7 DNAzyme against GATA-3mRNA
   <222> (1)..(33)
<400> 7
   gggcgacgag gctagctaca acgatctgca att 33
<210> 8
   <211> 33
   <212> DNA
   <213> Homo sapiens
<220>
   <221> hgd8 DNAzyme against GATA-3mRNA
   <222> (1)..(33)
<400> 8
   aaggggcgag gctagctaca acgagactct gca 33
<210> 9
   <211> 33
   <212> DNA
   <213> Homo sapiens
<220>
   <221> hgd9 DNAzyme against GATA-3mRNA
   <222> (1)..(33)
<400> 9
   aaaacgggag gctagctaca acgacaggtt gta 33
<210> 10
   <211> 33
   <212> DNA
   <213> Homo sapiens
<220>
   <221> hgd10 DNAzyme against GATA-3mRNA
   <222> (1)..(33)
<400> 10
   agaataaaag gctagctaca acgagggacc agg 33
<210> 11
   <211> 33
   <212> DNA
   <213> Homo sapiens
<220>
   <221> hgd11 DNAzyme against GATA-3mRNA
   <222> (1)..(33)
<400> 11
   atggcagaag gctagctaca acgaaaaacg gga 33
<210> 12
   <211> 33
   <212> DNA
   <213> Homo sapiens
<220>
   <221> hgd12 DNAzyme against GATA-3mRNA
   <222> (1)..(33)
<400> 12
   aactgggtag gctagctaca acgaggcaga ata 33
<210> 13
   <211> 33
   <212> DNA
   <213> Homo sapiens
<220>
   <221> hgd13 DNAzyme against GATA-3mRNA
   <222> (1)..(33)
<400> 13
   atccaaaaag gctagctaca acgatgggta tgg 33
<210> 14
   <211> 33
   <212> DNA
   <213> Homo sapiens
<220>
   <221> hgd14 DNAzyme against GATA-3mRNA
   <222> (1)..(33)
<400> 14
   aggggaagag gctagctaca acgaaaaaat cca 33
<210> 15
   <211> 33
   <212> DNA
   <213> Homo sapiens
<220>
   <221> hgd15 DNAzyme against GATA-3mRNA
   <222> (1)..(33)
<400> 15
   ttttaaaaag gctagctaca acgatatctt gga 33
<210> 16
   <211> 33
   <212> DNA
   <213> Homo sapiens
<220>
   <221> hgd16 DNAzyme against GATA-3mRNA
   <222> (1)..(33)
<400> 16
   gtggggggag gctagctaca acgagggaag gct 33
<210> 17
   <211> 33
   <212> DNA
   <213> Homo sapiens
<220>
   <221> hgd17 DNAzyme against GATA-3mRNA
   <222> (1)..(33)
<400> 17
   gttgaatgag gctagctaca acgattgctt tcg 33
<210> 18
   <211> 33
   <212> DNA
   <213> Homo sapiens
<220>
   <221> hgd18 DNAzyme against GATA-3mRNA
   <222> (1)..(33)
<400> 18
   gtcgttgaag gctagctaca acgagatttg ctt 33
<210> 19
   <211> 33
   <212> DNA
   <213> Homo sapiens
<220>
   <221> hgd19 DNAzyme against GATA-3mRNA
   <222> (1)..(33)
<400> 19
   ggcccggaag gctagctaca acgaccgcgc gcg 33
<210> 20
   <211> 33
   <212> DNA
   <213> Homo sapiens
<220>
   <221> hgd20 DNAzyme against GATA-3mRNA
   <222> (1)..(33)
<400> 20
   tcacctccag gctagctaca acgaggcctc ggc 33
<210> 21
   <211> 33
   <212> DNA
   <213> Homo sapiens
<220>
   <221> hgd21 DNAzyme against GATA-3mRNA
   <222> (1)..(33)
<400> 21
   ccgccgtcag gctagctaca acgactccat ggc 33
<210> 22
   <211> 33
   <212> DNA
   <213> Homo sapiens
<220>
   <221> hgd22 DNAzyme against GATA-3mRNA
   <222> (1)..(33)
<400> 22
   ggtggctcag gctagctaca acgaccagcg cgg 33
<210> 23
   <211> 33
   <212> DNA
   <213> Homo sapiens
<220>
   <221> hgd23 DNAzyme against GATA-3mRNA
   <222> (1)..(33)
<400> 23
   cgttgagcag gctagctaca acgaggcggg gtg 33
<210> 24
   <211> 33
   <212> DNA
   <213> Homo sapiens
<220>
   <221> hgd24 DNAzyme against GATA-3mRNA
   <222> (1)..(33)
<400> 24
   ccgcgtccag gctagctaca acgagtagga gtg 33
<210> 25
   <211> 33
   <212> DNA
   <213> Homo sapiens
<220>
   <221> hgd25 DNAzyme against GATA-3mRNA
   <222> (1)..(33)
<400> 25
   cagcgggtag gctagctaca acgatgcgcc gcg 33
<210> 26
   <211> 33
   <212> DNA
   <213> Homo sapiens
<220>
   <221> hgd26 DNAzyme against GATA-3mRNA
   <222> (1)..(33)
<400> 26
   gcacatccag gctagctaca acgactcctc cgg 33
<210> 27
   <211> 33
   <212> DNA
   <213> Homo sapiens
<220>
   <221> hgd27 DNAzyme against GATA-3mRNA
   <222> (1)..(33)
<400> 27
   aaaagcacag gctagctaca acgaccacct cct 33
<210> 28
   <211> 33
   <212> DNA
   <213> Homo sapiens
<220>
   <221> hgd28 DNAzyme against GATA-3mRNA
   <222> (1)..(33)
<400> 28
   taaaaagcag gctagctaca acgaatccac ctc 33
<210> 29
   <211> 33
   <212> DNA
   <213> Homo sapiens
<220>
   <221> hgd29 DNAzyme against GATA-3mRNA
   <222> (1)..(33)
<400> 29
   gaccgtcgag gctagctaca acgagttaaa aag 33
<210> 30
   <211> 33
   <212> DNA
   <213> Homo sapiens
<220>
   <221> hgd30 DNAzyme against GATA-3mRNA
   <222> (1)..(33)
<400> 30
   ttgccttgag gctagctaca acgacgtcga tgt 33
<210> 31
   <211> 33
   <212> DNA
   <213> Homo sapiens
<220>
   <221> hgd31 DNAzyme against GATA-3mRNA
   <222> (1)..(33)
<400> 31
   agggcgggag gctagctaca acgagtggtt gcc 33
<210> 32
   <211> 33
   <212> DNA
   <213> Homo sapiens
<220>
   <221> hgd32 DNAzyme against GATA-3mRNA
   <222> (1)..(33)
<400> 32
   tggccctgag gctagctaca acgacgagtt tcc 33
<210> 33
   <211> 33
   <212> DNA
   <213> Homo sapiens
<220>
   <221> hgd33 DNAzyme against GATA-3mRNA
   <222> (1)..(33)
<400> 33
   acctctgcag gctagctaca acgacgtggc cct 33
<210> 34
   <211> 33
   <212> DNA
   <213> Homo sapiens
<220>
   <221> hgd34 DNAzyme against GATA-3mRNA
   <222> (1)..(33)
<400> 34
   cggagggtag gctagctaca acgactctgc acc 33
<210> 35
   <211> 33
   <212> DNA
   <213> Homo sapiens
<220>
   <221> hgd35 DNAzyme against GATA-3mRNA
   <222> (1)..(33)
<400> 35
   ggcggcacag gctagctaca acgactggct ccc 33
<210> 36
   <211> 33
   <212> DNA
   <213> Homo sapiens
<220>
   <221> hgd36 DNAzyme against GATA-3mRNA
   <222> (1)..(33)
<400> 36
   cgggcggcag gctagctaca acgaacctgg ctc 33
<210> 37
   <211> 33
   <212> DNA
   <213> Homo sapiens
<220>
   <221> hgd37 DNAzyme against GATA-3mRNA
   <222> (1)..(33)
<400> 37
   agggatccag gctagctaca acgagaagca gag 33
<210> 38
   <211> 33
   <212> DNA
   <213> Homo sapiens
<220>
   <221> hgd38 DNAzyme against GATA-3mRNA
   <222> (1)..(33)
<400> 38
   gggtagggag gctagctaca acgaccatga agc 33
<210> 39
   <211> 33
   <212> DNA
   <213> Homo sapiens
<220>
   <221> hgd39 DNAzyme against GATA-3mRNA
   <222> (1)..(33)
<400> 39
   gggctgagag gctagctaca acgatccagg ggg 33
<210> 40
   <211> 33
   <212> DNA
   <213> Homo sapiens
<220>
   <221> hgd40 DNAzyme against GATA-3mRNA
   <222> (1)..(33)
<400> 40
   gtggatggag gctagctaca acgagtcttg gag 33
<210> 41
   <211> 33
   <212> DNA
   <213> Homo sapiens
<220>
   <221> hgd 41 DNAzyme against GATA-3mRNA
   <222> (1)..(33)
<400> 41
   cgtggtggag gctagctaca acgaggacgt ctt 33
<210> 42
   <211> 33
   <212> DNA
   <213> Homo sapiens
<220>
   <221> hgd 42 DNAzyme against GATA-3mRNA
   <222> (1)..(33)
<400> 42
   gggggtagag gctagctaca acgaggagag ggg 33
<210> 43
   <211> 33
   <212> DNA
   <213> Homo sapiens
<220>
   <221> hgd 43 DNAzyme against GATA-3mRNA
   <222> (1)..(33)
<400> 43
   ggaggaggag gctagctaca acgagaggcc ggg 33
<210> 44
   <211> 33
   <212> DNA
   <213> Homo sapiens
<220>
   <221> hgd44 DNAzyme against GATA-3mRNA
   <222> (1)..(33)
<400> 44
   gccccccgag gctagctaca acgaaaggag gag 33
<210> 45
   <211> 33
   <212> DNA
   <213> Homo sapiens
<220>
   <221> hgd45 DNAzyme against GATA-3mRNA
   <222> (1)..(33)
<400> 45
   ccggggagag gctagctaca acgagtcctt cgg 33
<210> 46
   <211> 33
   <212> DNA
   <213> Homo sapiens
<220>
   <221> hgd46 DNAzyme against GATA-3mRNA
   <222> (1)..(33)
<400> 46
   ggacagcgag gctagctaca acgagggtcc ggg 33
<210> 47
   <211> 33
   <212> DNA
   <213> Homo sapiens
<220>
   <221> hgd47 DNAzyme against GATA-3mRNA
   <222> (1)..(33)
<400> 47
   tggggtggag gctagctaca acgaagcgat ggg 33
<210> 48
   <211> 33
   <212> DNA
   <213> Homo sapiens
<220>
   <221> hgd48 DNAzyme against GATA-3mRNA
   <222> (1)..(33)
<400> 48
   cttgaggcag gctagctaca acgatctttc tcg 33
<210> 49
   <211> 33
   <212> DNA
   <213> Homo sapiens
<220>
   <221> hgd49 DNAzyme against GATA-3mRNA
   <222> (1)..(33)
<400> 49
   cacctggtag gctagctaca acgattgagg cac 33
<210> 50
   <211> 33
   <212> DNA
   <213> Homo sapiens
<220>
   <221> hgd50 DNAzyme against GATA-3mRNA
   <222> (1)..(33)
<400> 50
   gcaggggcag gctagctaca acgactggta ctt 33
<210> 51
   <211> 33
   <212> DNA
   <213> Homo sapiens
<220>
   <221> hgd51 DNAzyme against GATA-3mRNA
   <222> (1)..(33)
<400> 51
   ccagcttcag gctagctaca acgagctgtc ggg 33
<210> 52
   <211> 33
   <212> DNA
   <213> Homo sapiens
<220>
   <221> hgd52 DNAzyme against GATA-3mRNA
   <222> (1)..(33)
<400> 52
   gtgggacgag gctagctaca acgatccagc ttc 33
<210> 53
   <211> 33
   <212> DNA
   <213> Homo sapiens
<220>
   <221> hgd53 DNAzyme against GATA-3mRNA
   <222> (1)..(33)
<400> 53
   ggagtgggag gctagctaca acgagactcc agc 33
<210> 54
   <211> 33
   <212> DNA
   <213> Homo sapiens
<220>
   <221> hgd54 DNAzyme against GATA-3mRNA
   <222> (1)..(33)
<400> 54
   atgctgccag gctagctaca acgagggagt ggg 33
<210> 55
   <211> 33
   <212> DNA
   <213> Homo sapiens
<220>
   <221> hgd55 DNAzyme against GATA-3mRNA
   <222> (1)..(33)
<400> 55
   gggcggtcag gctagctaca acgagctgcc acg 33
<210> 56
   <211> 33
   <212> DNA
   <213> Homo sapiens
<220>
   <221> hgd56 DNAzyme against GATA-3mRNA
   <222> (1)..(33)
<400> 56
   gaggctccag gctagctaca acgaccaggg cgg 33
<210> 57
   <211> 33
   <212> DNA
   <213> Homo sapiens
<220>
   <221> hgd57 DNAzyme against GATA-3mRNA
   <222> (1)..(33)
<400> 57
   gtgggtcgag gctagctaca acgagaggag gct 33
<210> 58
   <211> 33
   <212> DNA
   <213> Homo sapiens
<220>
   <221> hgd58 DNAzyme against GATA-3mRNA
   <222> (1)..(33)
<400> 58
   aggtggtgag gctagctaca acgaggggtg gtg 33
<210> 59
   <211> 33
   <212> DNA
   <213> Homo sapiens
<220>
   <221> hgd59 DNAzyme against GATA-3mRNA
   <222> (1)..(33)
<400> 59
   actcgggcag gctagctaca acgagtaggg cgg 33
<210> 60
   <211> 33
   <212> DNA
   <213> Homo sapiens
<220>
   <221> hgd60 DNAzyme against GATA-3mRNA
   <222> (1)..(33)
<400> 60
   ggagctgtag gctagctaca acgatcgggc acg 33
<210> 61
   <211> 33
   <212> DNA
   <213> Homo sapiens
<220>
   <221> hgd61 DNAzyme against GATA-3mRNA
   <222> (1)..(33)
<400> 61
   ggacttgcag gctagctaca acgaccgaag ccg 33
<210> 62
   <211> 33
   <212> DNA
   <213> Homo sapiens
<220>
   <221> hgd62 DNAzyme against GATA-3mRNA
   <222> (1)..(33)
<400> 62
   gggcctggag gctagctaca acgattgcat ccg 33
<210> 63
   <211> 33
   <212> DNA
   <213> Homo sapiens
<220>
   <221> hgd63 DNAzyme against GATA-3mRNA
   <222> (1)..(33)
<400> 63
   tgtgctggag gctagctaca acgacgggcc ttg 33
<210> 64
   <211> 33
   <212> DNA
   <213> Homo sapiens
<220>
   <221> hgd64 DNAzyme against GATA-3mRNA
   <222> (1)..(33)
<400> 64
   gttcacacag gctagctaca acgatccctg cct 33
<210> 65
   <211> 33
   <212> DNA
   <213> Homo sapiens
<220>
   <221> hgd65 DNAzyme against GATA-3mRNA
   <222> (1)..(33)
<400> 65
   cagttcacag gctagctaca acgaactccc tgc 33
<210> 66
   <211> 33
   <212> DNA
   <213> Homo sapiens
<220>
   <221> hgd66 DNAzyme against GATA-3mRNA
   <222> (1)..(33)
<400> 66
   cacagttcag gctagctaca acgaacactc cct 33
<210> 67
   <211> 33
   <212> DNA
   <213> Homo sapiens
<220>
   <221> hgd67 DNAzyme against GATA-3mRNA
   <222> (1)..(33)
<400> 67
   gttgccccag gctagctaca acgaagttca cac 33
<210> 68
   <211> 33
   <212> DNA
   <213> Homo sapiens
<220>
   <221> hgd68 DNAzyme against GATA-3mRNA
   <222> (1)..(33)
<400> 68
   tcgccgccag gctagctaca acgaagtggg gtc 33
<211> 33
   <212> DNA
   <213> Homo sapiens
<220>
   <221> hgd69 DNAzyme against GATA-3mRNA
   <222> (1)..(33)
<400> 69
   cccgtgccag gctagctaca acgactcgcc gcc 33
<210> 70
   <211> 33
   <212> DNA
   <213> Homo sapiens
<220>
   <221> hgd70 DNAzyme against GATA-3mRNA
   <222> (1)..(33)
<400> 70
   ggcgttgcag gctagctaca acgaaggtag tgt 33
<210> 71
   <211> 2399
   <212> DNA
   <213> Homo sapiens
<400> 71
   ggcgccgtct tgatactttc agaaagaatg cattccctgt aaaaaaaaaa aaaaaatact 60
   gagagaggga gagagagaga gaagaagaga gagagacgga gggagagcga gacagagcga 120
   gcaacgcaat ctgaccgagc aggtcgtacg ccgccgcctc ctcctcctct ctgctcttcg 180
   ctacccaggt gacccgagga gggactccgc ctccgagcgg ctgaggaccc cggtgcagag 240
   gagcctggct cgcagaattg cagagtcgtc gccccttttt acaacctggt cccgttttat 300
   tctgccgtac ccagtttttg gatttttgtc ttccccttct tctctttgct aaacgacccc 360
   tccaagataa tttttaaaaa accttctcct ttgctcacct ttgcttccca gccttcccat 420
   ccccccaccg aaagcaaatc attcaacgac ccccgaccct ccgacggcag gagccccccg 480
   acctcccagg cggaccgccc tccctccccg cgcgcgggtt ccgggcccgg cgagagggcg 540
   cgagcacagc cgaggccatg gaggtgacgg cggaccagcc gcgctgggtg agccaccacc 600
   accccgccgt gctcaacggg cagcacccgg acacgcacca cccgggcctc agccactcct 660
   acatggacgc ggcgcagtac ccgctgccgg aggaggtgga tgtgcttttt aacatcgacg 720
   gtcaaggcaa ccacgtcccg ccctactacg gaaactcggt cagggccacg gtgcagaggt 780
   accctccgac ccaccacggg agccaggtgt gccgcccgcc tctgcttcat ggatccctac 840
   cctggctgga cggcggcaaa gccctgggca gccaccacac cgcctccccc tggaatctca 900
   gccccttctc caagacgtcc atccaccacg gctccccggg gcccctctcc gtctaccccc 960
   cggcctcgtc ctcctccttg tcggggggcc acgccagccc gcacctcttc accttcccgc 1020
   ccaccccgcc gaaggacgtc tccccggacc catcgctgtc caccccaggc tcggccggct 1080
   cggcccggca ggacgagaaa gagtgcctca agtaccaggt gcccctgccc gacagcatga 1140
   agctggagtc gtcccactcc cgtggcagca tgaccgccct gggtggagcc tcctcgtcga 1200
   cccaccaccc catcaccacc tacccgccct acgtgcccga gtacagctcc ggactcttcc 1260
   cccccagcag cctgctgggc ggctccccca ccggcttcgg atgcaagtcc aggcccaagg 1320
   cccggtccag cacagaaggc agggagtgtg tgaactgtgg ggcaacctcg accccactgt 1380
   ggcggcgaga tggcacggga cactacctgt gcaacgcctg cgggctctat cacaaaatga 1440
   acggacagaa ccggcccctc attaagccca agcgaaggct gtctgcagcc aggagagcag 1500
   ggacgtcctg tgcgaactgt cagaccacca caaccacact ctggaggagg aatgccaatg 1560
   gggaccctgt ctgcaatgcc tgtgggctct actacaagct tcacaatatt aacagacccc 1620
   tgactatgaa gaaggaaggc atccagacca gaaaccgaaa aatgtctagc aaatccaaaa 1680
   agtgcaaaaa agtgcatgac tcactggagg acttccccaa gaacagctcg tttaacccgg 1740
   ccgccctctc cagacacatg tcctccctga gccacatctc gcccttcagc cactccagcc 1800
   acatgctgac cacgcccacg ccgatgcacc cgccatccag cctgtccttt ggaccacacc 1860
   acccctccag catggtcacc gccatgggtt agagccctgc tcgatgctca cagggccccc 1920
   agcgagagtc cctgcagtcc ctttcgactt gcatttttgc aggagcagta tcatgaagcc 1980
   taaacgcgat ggatatatgt ttttgaaggc agaaagcaaa attatgtttg ccactttgca 2040
   aaggagctca ctgtggtgtc tgtgttccaa ccactgaatc tggaccccat ctgtgaataa 2100
   gccattctga ctcatatccc ctatttaaca gggtctctag tgctgtgaaa aaaaaaatgc 2160
   tgaacattgc atataactta tattgtaaga aatactgtac aatgacttta ttgcatctgg 2220
   gtagctgtaa ggcatgaagg atgccaagaa gtttaaggaa tatgggagaa atagtgtgga 2280
   aattaagaag aaactaggtc tgatattcaa atggacaaac tgccagtttt gtttcctttc 2340
   actggccaca gttgtttgat gcattaaaag aaaataaaaa aaagaaaaaa gagaaaaga 2399
<210> 72
   <211> 2365
   <212> DNA
   <213> Homo sapiens
<400> 72
   tcccagcctt cccatccccc caccgaaagc aaatcattca acgacccccg accctccgac 60
   ggcaggagcc ccccgacctc ccaggcggac cgcccttccc tccccgcgcg ggttccgggc 120
   ccggcgagag ggcgcgacga cagccgaggc catggaggtg acggcggacc agccgcgctg 180
   ggtgagccac caccaccccg ccgtgctcaa cgggcagcac ccggacacgc accacccggg 240
   cctcagccac tcctacatgg acgcggcgca gtacccgctg ccggaggagg tggatgtgct 300
   ttttaacatc gacggtcaag gcaaccacgt cccgccctac tacggaaact cggtcagggc 360
   cacggtgcag aggtaccctc cgacccacca cgggagccag gtgtgccgcc cgcctctgct 420
   tcatggatcc ctaccctggc tggacggcgg caaagccctg ggcagccacc acaccgcctc 480
   cccctggaat ctcagcccct tctccaagac gtccatccac cacggctccc cggggcccct 540
   ctccgtctac cccccggcct cgtcctcctc cttgtcgggg ggccacgcca gcccgcacct 600
   cttcaccttc ccgcccaccc cgccgaagga cgtctccccg gacccatcgc tgtccacccc 660
   aggctcggcc ggctcggccc ggcaggacga gaaagagtgc ctcaagtacc aggtgcccct 720
   gcccgacagc atgaagctgg agtcgtccca ctcccgtggc agcatgaccg ccctgggtgg 780
   agcctcctcg tcgacccacc accccatcac cacctacccg ccctacgtgc ccgagtacag 840
   ctccggactc ttccccccca gcagcctgct gggcggctcc cccaccggct tcggatgcaa 900
   gtccaggccc aaggcccggt ccagcacagg cagggagtgt gtgaactgtg gggcaacctc 960
   gaccccactg tggcggcgag atggcacggg acactacctg tgcaacgcct gcgggctcta 1020
   tcacaaaatg aacggacaga accggcccct cattaagccc aagcgaaggc tgtctgcagc 1080
   caggagagca gggacgtcct gtgcgaactg tcagaccacc acaaccacac tctggaggag 1140
   gaatgccaat ggggaccctg tctgcaatgc ctgtgggctc tactacaagc ttcacaatat 1200
   taacagaccc ctgactatga agaaggaagg catccagacc agaaaccgaa aaatgtctag 1260
   caaatccaaa aagtgcaaaa aagtgcatga ctcactggag gacttcccca agaacagctc 1320
   gtttaacccg gccgccctct ccagacacat gtcctccctg agccacatct cgcccttcag 1380
   ccactccagc cacatgctga ccacgcccac gccgatgcac ccgccatcca gcctgtcctt 1440
   tggaccacac cacccctcca gcatggtcac cgccatgggt tagagccctg ctcgatgctc 1500
   acagggcccc cagcgagagt ccctgcagtc cctttcgact tgcatttttg caggagcagt 1560
   atcatgaagc ctaaacgcga tggatatatg tttttgaagg cagaaagcaa aattatgttt 1620
   gccactttgc aaaggagctc actgtggtgt ctgtgttcca accactgaat ctggacccca 1680
   tctgtgaata agccattctg actcatatcc cctatttaac agggtctcta gtgctgtgaa 1740
   aaaaaaaaat cctgaacatt gcatataact tatattgtaa gaaatactgt acaatgactt 1800
   tattgcatct gggtagctgt aaggcatgaa ggatgccaag aagtttaagg aatatgggag 1860
   aaatagtgtg gaaattaaga agaaactagg tctgatattc aaatggacaa actgccagtt 1920
   ttgtttcctt tcactggcca cagttgtttg atgcattaaa agaaaataaa aaaaagaaaa 1980
   aagagaaaag aaaaaaaaag aaaaaagttg taggcgaatc atttgttcaa agctgttggc 2040
   cctctgcaaa ggaaatacca gttctgggca atcagtgtta ccgttcacca gttgccattg 2100
   agggtttcag agagcctttt tctaggccta catgctttgt gaacaagtcc ctgtaattgt 2160
   tgtttgtatg tataattcaa agcaccaaaa taagaaaaga tgtagattta tttcatcata 2220
   ttatacagac cgaactgttg tataaattta tttactgcta gtcttaagaa ctgctttctt 2280
   tcgtttgttt gtttcaatat tttccttctc tctcaatttt cggttgaata aactagatta 2340
   cattcagttg gcaaaaaaaa aaaaa 2365
<210> 73
   <211> 2728
   <212> DNA
   <213> Homo sapiens
<220>
   <221> mutation
   <222> (57)..(57)
<220>
   <221> mutation
   <222> (59)..(59)
<220>
   <221> mutation
   <222> (69)..(69)
<220>
   <221> hgd40 bindingsite
   <222> (909)..(927)
<400> 73
   ggcgccgtct tgatactttc agaaagaatg cattccctgt aaaaaaaaaa aaaaaaaaat 60
   actgagagag ggagagagag agaagaagag agagagacgg agggagagcg agacagagcg 120
   agcaacgcaa tctgaccgag caggtcgtac gccgccgcct cctcctcctc tctgctcttc 180
   gctacccagg tgacccgagg agggactccg cctccgagcg gctgaggacc ccggtgcaga 240
   ggagcctggc tcgcagaatt gcagagtcgt cgcccctttt tacaacctgg tcccgtttta 300
   ttctgccata cccagttttt ggatttttgt cttccccttc ttctctttgc taaacgaccc 360
   ctccaagata atttttaaaa aaccttctcc tttgctcacc tttgcttccc agccttccca 420
   tccccccacc gaaagcaaat cattcaacga cccccgaccc tccgacggca ggagcccccc 480
   gacctcccag gcggaccgcc ctccctcccc gcgcgcgggt tccgggcccg gcgagagggc 540
   gcgagcacag ccgaggccat ggaggtgacg gcggaccagc cgcgctgggt gagccaccac 600
   caccccgccg tgctcaacgg gcagcacccg gacacgcacc acccgggcct cagccactcc 660
   tacatggacg cggcgcagta cccgctgccg gaggaggtgg atgtgctttt taacatcgac 720
   ggtcaaggca accacgtccc gccctactac ggaaactcgg tcagggccac ggtgcagagg 780
   taccctccga cccaccacgg gagccaggtg tgccgcccgc ctctgcttca tggatccctc 840
   cctggctgga cggcggcaaa gccctgggca gccaccacac cgcctccccc tggaatctca 900
   gccccttctc caagacgtcc atccaccacg gctccccggg gcccctctcc gtctaccccc 960
   cggcctcgtc ctcctccttg tcggggggcc acgccagccc gcacctcttc accttcccgc 1020
   ccaccccgcc gaaggacgtc tccccggacc catcgctgtc caccccaggc tcggccggct 1080
   cggcccggca ggacgagaaa gagtgcctca agtaccaggt gcccctgccc gacagcatga 1140
   agctggagtc gtcccactcc cgtggcagca tgaccgccct gggtggagcc tcctcgtcga 1200
   cccaccaccc catcaccacc tacccgccct acgtgcccga gtacagctcc ggactcttcc 1260
   cccccagcag cctgctgggc ggctccccca ccggcttcgg atgcaagtcc aggcccaagg 1320
   cccggtccag cacagaaggc agggagtgtg tgaactgtgg ggcaacctcg accccactgt 1380
   ggcggcgaga tggcacggga cactacctgt gcaacgcctg cgggctctat cacaaaatga 1440
   acggacagaa ccggcccctc attaagccca agcgaaggct gtctgcagcc aggagagcag 1500
   ggacgtcctg tgcgaactgt cagaccacca caaccacact ctggaggagg aatgccaatg 1560
   gggaccctgt ctgcaatgcc tgtgggctct actacaagct tcacaatatt aacagacccc 1620
   tgactatgaa gaaggaaggc atccagacca gaaaccgaaa aatgtctagc aaatccaaaa 1680
   agtgcaaaaa agtgcatgac tcactggagg acttccccaa gaacagctcg tttaacccgg 1740
   ccgccctctc cagacacatg tcctccctga gccacatctc gcccttcagc caccccagcc 1800
   acatgctgac cacgcccacg ccgatgcacc cgccatccag cctgtccttt ggaccacacc 1860
   acccctccag catggtcacc gccatgggtt agagccctgc tgatgctcac agggccccca 1920
   gcgagagtcc ctgcagtccc tttcgacttg catttttgca ggagcagtat catgaagcct 1980
   aaacgcgatg gatatatgtt tttgaaggca gaaagcaaaa ttatgcttgc cactttgcaa 2040
   aggagctcac tgtggtgtct gtgttccaac cactgaatct ggaccccatc tgtgaataag 2100
   ccattctgac tcatatcccc tatttaacag ggtctctagt gctgtgaaaa aaaaaaatgc 2160
   tgaacattgc atataactta tattgtaaga aatactgtac aatgacttta ttgcatctgg 2220
   gtagctgtaa ggcatgaagg atgccaagaa gtttaaggaa tatgggagaa atagtgtgga 2280
   aattaagaag aaactaggtc tgatattcaa atggacaaac tgccagtttt gtttcctttc 2340
   actggccaca gttgtttgat gcattaaaag aaaataaaaa aaagaaaaag agaaaagaaa 2400
   aaaaaagaaa aaagttgtag gcgaatcatt tgttcaaagc tgttggcctc tgcaaaggaa 2460
   ataccagttc gggcaatcag tgttaccgtt caccagttgc cattgagggt ttcagagagc 2520
   ctttttctag gcctacatgc tttgtgaaca agtccctgta attgttgttt gtatgtataa 2580
   ttcaaagcac caaaataaga aaagatgtag atttatttca tcatattata cagaccgaac 2640
   tgttgtataa atttatttac tgctagtctt aagaactgct ttctttcgtt tgtttgtttc 2700
   aatattttcc ttctctctca attttcgg 2728